# EUROPEAN PATENT SPECIFICATION

(11) **EP 4 192 975 B1**
(45) Date of publication and mention of the grant of the patent: **10.07.2024**
(21) Application number: 21814970.6
(22) Date of filing: 19.10.2021
(51) Int. Cl.: C12Q 1/60, G01N 33/49, G01N 33/52, G01N 33/53, G01N 33/543, G01N 33/558, G01N 33/92

(54) **ASSAY DEVICE AND METHOD FOR CONCURRENT SEPARATION OF RED BLOOD CELLS AND A PROTEIN FROM A BLOOD FLUID SAMPLE**
ASSAY-VORRICHTUNG UND VERFAHREN ZUR GLEICHZEITIGEN TRENNUNG VON ROTEN BLUTZELLEN UND EINEM PROTEIN AUS EINER BLUTFLÜSSIGKEITSPROBE
DISPOSITIF ET PROCÉDÉ POUR LA SÉPARATION SIMULTANÉE DES GLOBULES ROUGES ET D'UNE PROTÉINE À PARTIR D'UN ÉCHANTILLON DE FLUIDE SANGUIN

(43) Date of publication of application: 14.06.2023
(73) Proprietor: Google LLC, Mountain View, CA 94043 (US)
(72) Inventor: Watkins, Herschel, Mountain View, CA 94043 (US); McKeating, Kristy, Mountain View, CA 94043 (US)
(74) Representative: Maikowski & Ninnemann Patentanwälte Partnerschaft mbB
(86) International application number: PCT/US2021/055565
(87) International publication number: WO 2023/069075

(56) References cited:
- EP-A1- 0 408 223
- EP-A2- 2 009 447
- WO-A1-2004/090555
- US-A- 5 786 164
- US-A1- 2014 255 960

## Description

### FIELD

The present disclosure relates generally to a point-of-care (POC) testing system that includes an assay device. More particularly, the present disclosure relates to assay devices and methods for concurrently separating red blood cells and a first protein from a second protein in a single separation membrane.

### BACKGROUND

Assay devices for performing medical diagnostic tests are for example disclosed in EP 2 009 447 A2, EP 0 408 223 A1, US 5,786,164 A and WO 2004/090555 A1. Point-of-care (POC) testing refers to performing medical diagnostic tests at the time and place that the patient is being treated. POC testing is advantageous over traditional diagnostic testing where patient samples are sent out to a laboratory for further analysis, because the results of traditional diagnostic tests may not be available for hours, if not days or weeks, making it difficult for a caregiver to assess the proper course of treatment in the interim.

Of particular interest in POC testing is the determination of the level of cholesterol present in a blood fluid sample, where the level of cholesterol may be useful in assessing a patient's risk for coronary artery disease. A measure of total cholesterol includes low density lipoproteins (LDL), very low density lipoproteins (VLDL), hereinafter collectively referred to as non-HDL, and high density lipoproteins (HDL). It is generally known that there is a positive correlation between LDL and VLDL values and coronary artery disease and a negative correlation between HDL values and coronary artery diseases. Thus, LDL and VLDL are often referred to as "bad cholesterol" while HDL is often referred to as "good cholesterol." Due to this distinction, there is a benefit to determining the amount of HDL in a blood fluid sample to determine a patient's risk of heart disease. This requires separating the HDL from other proteins and components (e.g., red blood cells or erythrocytes) in a blood fluid sample in order to arrive at an accurate reading of the HDL present. For instance, the red blood cells are typically separated first, followed by the separation of non-HDL from the blood fluid sample. Thereafter, the remaining portion of the blood fluid sample is analyzed to determine the amount of HDL present. This process requires relatively large blood volumes, as a portion of blood is lost with each separation step.

Many commercially available assays solve this problem by using several separation phases in immediate physical proximity. The layers typically include a spreading mesh, a plasma separation layer, an HDL separation layer, and an analysis or detection layer. This four-layer approach requires a large volume of blood that is not feasible for POC devices utilizing a comfortable finger lancet volume.

Thus, it would be desirable to have a POC system that can determine an amount of HDL present in a blood fluid sample without having to utilize as many layers, which allows for a reduced sample blood volume and a more efficient assay process.

### SUMMARY

The proposed solution relates to an assay device of claim 1 and a method of claim 13.

Aspects and advantages of embodiments of the present disclosure will be set forth in part in the following description, or can be learned from the description, or can be learned through practice of the embodiments.

One example aspect of the present disclosure is directed to an assay device for analyzing a blood fluid sample. The assay device includes a separation membrane and a detection membrane. The separation membrane contains a first reagent for precipitating a first protein contained in the blood fluid sample, where the separation membrane also separates red blood cells from the blood fluid sample and the first reagent comprises a polyanionic non-high density lipoprotein binding reagent. Further, the detection membrane is configured to elicit a quantifiable response in the presence of a second protein present in the blood fluid sample. The quantifiable response corresponds to an amount of the second protein present in the detection membrane. In addition, it is to be understood that the separation membrane concurrently precipitates the first protein and separates the red blood cells from the blood fluid sample that reaches the detection membrane, such that, for example, the blood fluid sample is free of the first protein and the red blood cells when the blood fluid sample reaches the detection membrane and is analyzed for the presence of the second protein. In the proposed solution the first reagent is present in the separation membrane at (a) a concentration ranging from about 35 milligrams/milliliter to about 250 milligrams/milliliter based on the wet weight of the first reagent present on the separation membrane or (b) a concentration ranging from about 0.7 milligrams/square centimeter to about 10 milligrams/square centimeter based on the dry weight of the first reagent present on the separation membrane.

Another aspect of the present disclosure is directed to an in-vitro use of the proposed assay device for determining the concentration of the second protein present in the blood fluid sample.

Yet another aspect of the present disclosure is directed to a use of the assay device in a diagnostic method for determining the concentration of the second protein present in the blood fluid sample.

Still another aspect of the present disclosure is directed to a method of fabricating an assay device for analyzing a blood fluid sample. The method includes, in no particular order, the steps of: applying a first reagent comprising a polyanionic non-high density lipoprotein binding reagent to a separation membrane, wherein the first reagent precipitates a first protein contained in the blood fluid sample when the blood fluid sample contacts the separation membrane, further wherein the separation membrane is configured to separate red blood cells from the blood fluid sample; allowing the first reagent to dry on the separation membrane; and positioning a detection membrane adj acent the separation membrane, wherein the detection membrane is configured to elicit a quantifiable response in the presence of a second protein present in the blood fluid sample, wherein the quantifiable response corresponds to an amount of the second protein present in the detection membrane, wherein the separation membrane is configured to concurrently precipitate the first protein and separate the red blood cells from the blood fluid sample that reaches the detection membrane. The first reagent is present in the separation membrane at (a) a concentration ranging from about 35 milligrams/milliliter to about 250 milligrams/milliliter based on the wet weight of the first reagent present on the separation membrane or (b) a concentration ranging from about 0.7 milligrams/square centimeter to about 10 milligrams/square centimeter based on the dry weight of the first reagent present on the separation membrane.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1 provides a schematic drawing of a system comprising a cartridge and an assay reader according to one embodiment of the disclosure;
FIGs. 2A-2C illustrate an embodiment of the cartridge utilized in the system;
FIG. 3 illustrates various layers of a metering stack contained within the cartridge;
FIG. 4 illustrates various layers of an assay stack contained within the cartridge;
FIG. 5A shows a longitudinal cross sectional view of an assay reader according to one embodiment of the disclosure;
FIG. 5B shows a longitudinal cross sectional view of an assay reader with an inserted cartridge according to one embodiment of the disclosure;
FIG. 6A shows a transverse cross-sectional view of an assay reader according to one embodiment of the disclosure;
FIG. 6B shows a transverse cross sectional view of the assay reader with an inserted cartridge according to one embodiment of the disclosure;
FIG. 7 shows a block diagram of the sensor system of the assay reader, according to an exemplary implementation of the disclosure;
FIGs. 8A-8D illustrate a cartridge that includes a metering stack and an assay stack during various stages of the assay process after a blood fluid sample has been introduced to the cartridge;
FIG. 9 shows a flow chart illustrating a method of using the assay system according to an exemplary implementation of the disclosure;
FIG. 10 shows a flow chart illustrating a method of manufacturing a cartridge according to one exemplary implementation of the disclosure;
FIG. 11 shows a graph of high density lipoprotein concentration versus signal (Kubelka-Munk intensity or KM) expressed by a reagent for high density lipoprotein in a detection membrane when the separation membrane contained varying levels of a first reagent for precipitating the non-high density lipoproteins in a blood fluid sample for a manually assembled assay stack;
FIG. 12 shows a graph of total cholesterol concentration versus signal (Kubelka-Munk intensity or KM) expressed by a reagent for total cholesterol in a detection membrane when the separation membrane contained varying levels of a first reagent for precipitating the non-high density lipoproteins in a blood fluid sample for a manually assembled assay stack;
FIG. 13 shows a graph of high density lipoprotein concentration versus signal (Kubelka-Munk intensity or KM) expressed by a reagent for high density lipoprotein in a detection membrane when the separation membrane contained varying levels of a first reagent for precipitating the non-high density lipoproteins in a blood fluid sample for a manufactured assay stack;
FIG. 14 shows a graph of total cholesterol concentration versus signal (Kubelka-Munk intensity or KM) expressed by a reagent for total cholesterol in a detection membrane when the separation membrane contained varying levels of a first reagent for precipitating the non-high density lipoproteins in a blood fluid sample for a manufactured assay stack; and
FIG. 15 shows a graph of residual non-high density lipoprotein concentration versus signal (Kubelka-Munk intensity or KM) expressed by a reagent for non-high density lipoprotein in a detection membrane when the separation membrane contained varying levels of the reagent for precipitating the non-high density lipoproteins in a blood fluid sample for a manufactured assay stack.

Reference numerals that are repeated across plural figures are intended to identify the same features in various implementations.

### DETAILED DESCRIPTION

Any of the features, components, or details of any of the arrangements or embodiments disclosed in this application, including without limitation any of the cartridge embodiments and any of the testing or assay embodiments disclosed below, are interchangeably combinable with any other features, components, or details of any of the arrangements or embodiments disclosed herein to form new arrangements and embodiments.

Generally, the present disclosure is related to an assay device for the concurrent separation of red blood cells and a first protein in a blood fluid sample for subsequent analysis for the presence of a second protein in the blood fluid sample. The assay device accomplishes such concurrent separation using a single separation layer. The present disclosure also provides methods for using the assay device to separate the red blood cells and the first protein in order to analyze the blood fluid sample to quantify the level of the second protein that is present in the blood fluid sample.

In particular, the assay device can be in the form of a cartridge that includes an assay stack. The assay stack includes a separation membrane containing a first reagent for precipitating a first protein contained in the blood flood sample. Further, the separation layer can have a pore size ranging from about 0.1 micrometers to about 2 micrometers. The assay device also includes a detection membrane. The detection membrane is configured to elicit a quantifiable response in the presence of a second protein present in the blood fluid sample. Further, the quantifiable response corresponds to an amount of the second protein present in the detection membrane. In addition, the separation prevents red blood cells and the first protein from entering the detection membrane. In one embodiment, the first protein is a non-high density lipoprotein and the second protein is a high density lipoprotein.

Unlike other assay devices, which separate red blood cells from the blood fluid sample in a first layer, followed by the separation of non-high density lipoproteins in a second layer, which requires large blood volumes, the present disclosure contemplates concurrent separation of the red blood cells and the non-high density lipoproteins. This requires a reduced blood fluid sample volume compared to traditional commercially available assay devices. In such devices, a spreading mesh, a plasma separation layer, and a non-high density lipoprotein separation layer, and a high density lipoprotein detection layer or analysis layer are required. In contrast, the assay device and methods of the present disclosure only require a separation membrane or layer and a detection membrane or layer.

The proposed solution allows for providing a compact POC testing system with a proposed assay device capable of an in-vitro assay of an isolated blood fluid sample with concurrent separation of a first protein (e.g., non-HDL) and red blood cells without the need for additional layers or membranes in the POC system to carry out such separation from the blood fluid sample to be analyzed for a target analyte of interest (e.g., a second protein such as HDL). A cartridge constructed by an embodiment of a proposed assay device with an assay stack comprising only a separation membrane and a detection membrane in this context allows for a cost-efficient design and requires less blood fluid sample volume compared to conventional HDL assay systems. In combination with an assay reader configured to be compatible with a proposed assay device so that the proposed assay device can be inserted into the assay ready for distributing a blood sample to the separation membrane, the detection of the target analyte may be automated in an easy way, since any target analyte present in the blood fluid sample may flow through the assay stack from the separation membrane to the detection membrane, while any interfering or unwanted blood components are left behind in the separation membrane. Based on the detection membrane allowing for qualitatively or quantitatively determining the amount of target analyte in the blood sample fluid based on a resulting signal (e.g., a color change) in the presence of a reagent for the target analyte, an automated assessment on the presence of the target analyte in the blood fluid sample is possible.

With reference now to the figures, example embodiments of the present disclosure will be discussed in further detail. First, the components of the cartridge and assay reader will be discussed, followed by the components used to perform an assay as contemplated by the present disclosure, which can include a non-HDL precipitation reagent combined with an enzymatic cascade.

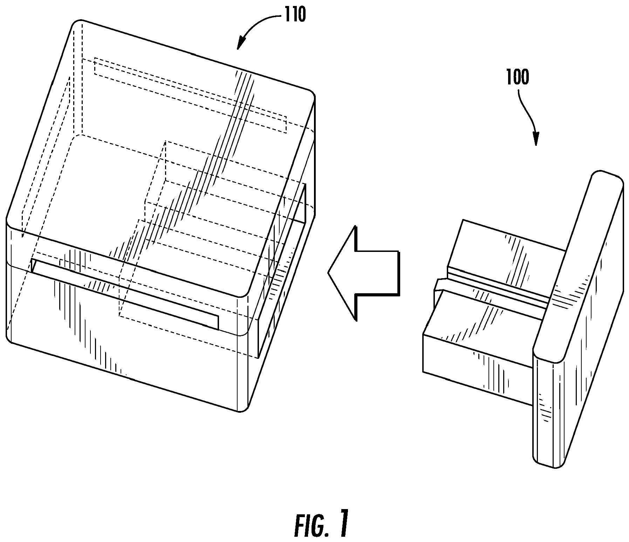

FIG. 1 shows a point-of-care (POC) testing system according to one exemplary embodiment of the present disclosure. The POC testing system includes a cartridge 100 and an assay reader 110. As described herein, cartridge 100 is used to collect the blood fluid sample that may potentially contain a target analyte such as a first protein (e.g., any non-HDL proteins). The collection process also distributes the blood fluid sample within cartridge 100. After the blood fluid sample is collected in cartridge 100, the user inserts cartridge 100 into assay reader 110. As described herein, the act of inserting cartridge 100 into assay reader 110 results in the compression of cartridge 100, thereby causing the blood fluid sample to be distributed to a separation membrane portion of an assay stack, the details of which are discussed in more detail below. In this way, the act of inserting cartridge 100 into assay reader 110 commences one or more assay reactions that provide information regarding the contents of the blood fluid sample. However, it is also to be understood that other insertion approaches are contemplated that do not require compression. Further, it is to be understood that while multiple assays can be utilized to determine the contents of a target analyte in the blood fluid sample, each assay is generally specific for one particular target analyte. As described herein, assay reader 110 is equipped with a detection system that is used to detect the results of the one or more assay reactions that occur at one or more membranes in an assay stack of cartridge 100. The detection system is not particularly limited and may be a detection system which causes a measurable signal change as the result of an assay reaction. Non-limiting examples of suitable detection systems include colorimetric, fluorescence, electrochemical, and optical detection systems as described herein and any other detection system that would be understood by one of ordinary skill in the art.

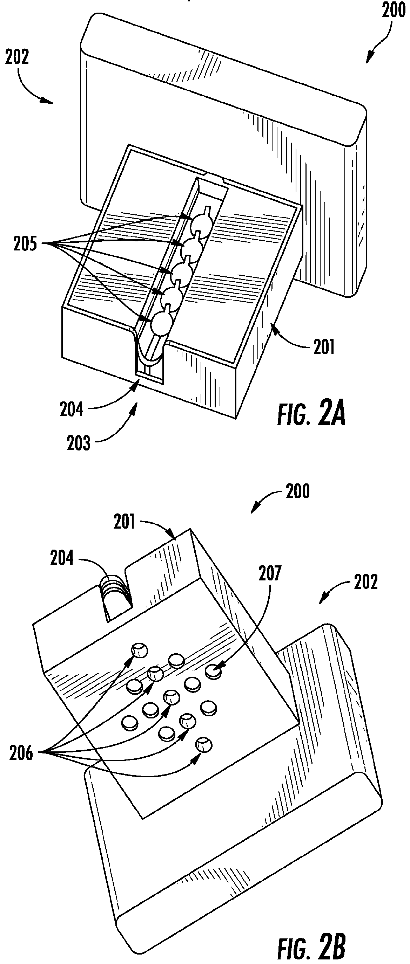

FIG. 2A illustrates a top, perspective view of an embodiment of cartridge 100 in the form of a cartridge 200. In FIG. 2A, cartridge 200 includes a housing 201 attached to a handle 202. In general, cartridge 200 is designed to be easy to handle by the user and to provide a protective shell for the microfluidic distribution system and assay components housed within cartridge 200. In general, suitable materials for housing 201 and handle 202 include polyolefinic compounds, such as polyethylene, polypropylene, and other polymeric resins or compounds known in the medical device manufacturing art. During sample collection, cartridge 200 is brought into contact with a fluid sample (e.g., the blood fluid sample). The fluid sample is drawn into channel 203 and via channel opening 204 by capillary action. In some embodiments, channel 203 includes a plurality of receiving chambers 205 located along channel 203. In some embodiments, each receiving chamber can be positioned between two venting holes, which facilitate the division of the fluid sample in the channel into multiple aliquots which flow to the assay stack. It should be recognized that the channel opening 204 can function as a venting hole and that neighboring receiving chambers can share a common venting hole between them. The venting holes can help to prevent unwanted bubble formation as the blood fluid sample is drawn into the receiving chambers. FIG. 2B illustrates a bottom view of an embodiment of the cartridge 200. In FIG. 2B, the bottom portion of housing 201 includes a plurality of assay detection ports 206 aligned with channel opening 204. The assay detection ports 206 permit the assay results to be interrogated, for example, by optical detection methods as described herein. In addition, the bottom portion of housing 201 may comprise plurality of holes 207, which are additional assay detection ports that may be used with assay components and microfluidic channels that are arranged in a corresponding configuration.

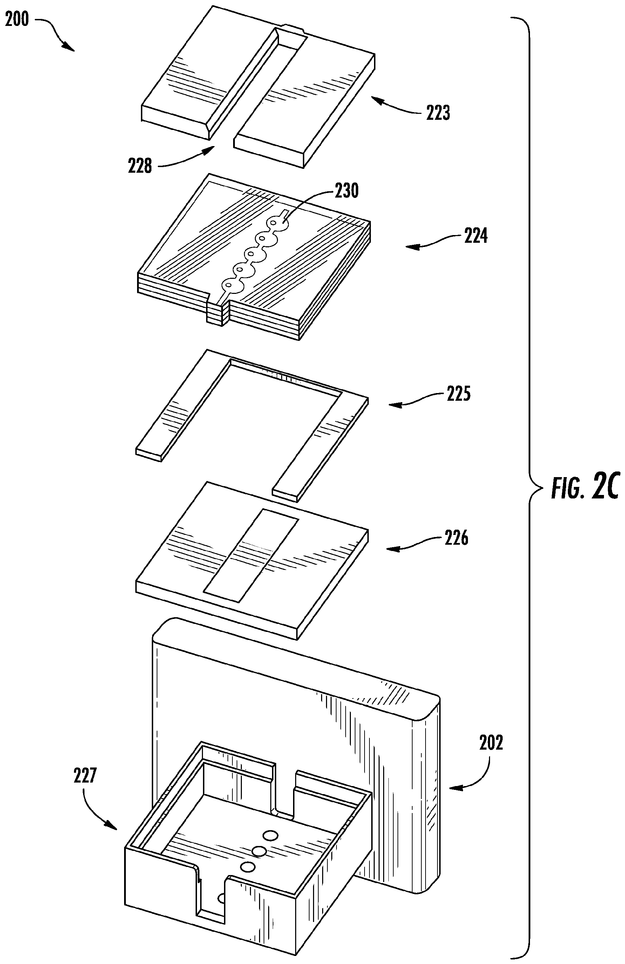

FIG. 2C provides an exploded view of the components of the cartridge 200, according to one embodiment of the present disclosure. In FIG. 2C, the outer shell of cartridge 200 includes the handle 202, bottom housing portion 227, and a cap 223 that is equipped with a slot 228. The bottom housing portion 227 can be a cuboid shape enclosure with one open side. The enclosure shape of the bottom housing portion 227 protects the components within the interior chamber and can avoid accidental actuation of the system. The cap 223 can fit to the open side of the bottom housing portion 227 and have a shape and size that corresponds to the open side of the bottom housing portion 227. When the bottom housing portion 227 and cap 223 of the housing are assembled together, an interior chamber can be formed for enclosing other components of the cartridge within the interior chamber. In other embodiments, the cap 223 and bottom housing portion 227 do not form an enclosure with an interior chamber and can be rigid structures positioned on the top of a metering stack and bottom of an assay stack, which are described herein.

In preferred embodiments, bottom housing portion 227 and cap 223 can be formed of a material to provide a rigid structure to the cartridge 200. For example, the bottom housing portion 227 and the cap 223 can be a plastic material, as described herein. The bottom housing portion 227 and cap 223 can be moveable or non-moveable with relation to each other. In some embodiments, when cartridge 200 is inserted into an assay reader, the components within the interior chamber are compressed to cause at least one portion of the collected blood fluid sample to be delivered to a plurality of assay components. The compression can be caused by the user closing a lid of the assay reader, for example. However, it is also to be understood that other approaches for insertion of the cartridge 200 into an assay reader are contemplated that do not require compression.

In some embodiments, the cartridge does not include a cap and bottom housing portion. In such embodiments, the cartridge does not include the housing 201 (see e.g., FIG. 2A) and the metering stack and assay stack can be inserted into an assay reader without an enclosure around it.

As shown in FIG. 2C, cartridge 200 can include a metering stack 224, a spacer material 225, and an assay stack 226. The metering stack 224 can be used to collect a sample of a biological fluid (e.g., a blood fluid sample) and the assay stack 226 includes assay components necessary for a protein concentration assay to be carried out through one or more of the receiving chambers 205 as discussed in detail herein. However, it should also be understood that other assays (e.g., immunoassays or enzymatic assays) may also be carried out in additional receiving chambers 205 and the assay stacks 226 associated with each receiving chamber 205 can each be unique based on the particular assay associated with each receiving chamber 205. As used herein, the term "metering" refers to collecting a liquid sample of a biological fluid and delivering one or more predetermined volumes of at least a portion of the fluid to the assay components for further analysis via the assay components contained in the assay stack. When assembled into a cartridge, the metering stack 224, a spacer material 225, and an assay stack 226 can be arranged in a stack.

The spacer material 225 is a compressible layer that may be positioned between the metering stack 224 and assay stack 226 as shown in FIG. 2C. In an embodiment, the spacer material 225 may be a flexible material that can be compressed in the vertical direction when the cartridge is inserted into the assay reader and the metering stack 224 is moved into contact with or close proximity to the assay stack 226. In some embodiments, the spacer material 225 can be a flexible material, such as foam, rubber, porous polymer, metal, cotton, or other bending, folding, or moving mechanisms such as a clamp or spring. In some embodiments, the metering and assay stacks are initially separated by an air gap maintained by the spacer material 225. In certain embodiments, spacer material 225 is physically affixed to another layer, such as metering stack 224 or assay stack 226 before the layers of the cartridge are brought together. Typically, the metering and assay stacks remain separated throughout the sample collection process. In such embodiments, the separation between the metering stack and the assay stack can prevent a chemical reaction from starting during the blood fluid sample collection step. When the spacer material 225 is compressed, the metering stack 224 and assay stack 226 can come into contact with or brought into close proximity to each other.

In preferred embodiments, when the metering stack is fully filled with a biological fluid, the cartridge is inserted into an assay reader. Preferably, the material that is used for the top surface of channel 230 is sufficiently transparent so that a user can determine by visual inspection when the channel 230 is filled and the cartridge is ready for insertion into the assay reader. The assay reader is configured to accept the cartridge and includes a mechanism that compresses the spacer material, thereby pushing the metering stack and assay stack together when the cartridge is inserted into the assay reader. The compression of the spacer material causes a predetermined volume of at least a portion of the collected fluid to flow to assay components in the assay stack. In this way, the act of compressing the metering stack and assay stack together can, in certain embodiments, provide a well-defined point in time that marks the start of the assay through the components in the assay stack. However, it is also to be understood that other insertion approaches are contemplated that do not require compression of the metering stack and assay stack together as would be understood by one of ordinary skill in the art.

In some embodiments, the fluid sample containing the target analyte is blood, and the cartridge can be used to collect a sample of blood from a skin prick and deliver the sample to the assay stack consistently with minimal user intervention. The user, with a regular pricking lancet, can elicit bleeding in a suitable body site such as a fingertip, palm, hand, forearm, stomach area, etc. Once a drop of blood of sufficient volume is on the skin, the user can collect it by touching the tip of the cartridge to the blood drop. Once the metering stack is fully filled with blood, the user can insert the cartridge into the assay reader, which triggers the delivery of the blood sample to the assay stack. In some embodiments, this can be performed by a patient, administrator, or healthcare provider. The blood collection and testing as described herein does not have to be performed by a trained heath care professional.

In addition, the cartridge design can allow for dispensing different pre-defined volumes of blood sample to multiple assay locations, without using any moving parts such as pumps or valves in the cartridge or in the assay reader. This increases the accuracy and flexibility of a multiplexed quantitative POC analysis, while reducing the complexity and cost of the cartridge and the assay reader.

Typically, as illustrated in FIG. 2C, the metering stack 224 includes a channel 230 to contain the biological fluid (e.g., a blood fluid sample containing a target analyte). In certain embodiments, the channel 230 can hold a volume of biological fluid containing a target analyte in the range of about 0.5 µl to about 100 µl, such as about 5 µl to about 80 µl, such as about 10 to about 60 µl, such as about 15 µl to about 40 µl, such about 20 µl to about 30 µl. The volume of the biological fluid can be controlled by the dimensions of the channel, including the shape, width, length, and depth of the channel, as described herein. In some embodiments, the depth of the channel can be in the range of about 5 µm to about 3 mm, about 10 µm to about 2 mm, or about 250 µm to about 1 mm. In some embodiments, the width of the channel can be in the range of about 100 µm to about 10 mm, about 250 µm to about 5 mm, about 500 µm to about 3 mm, or about 750 µm to about 1 mm. In certain preferred embodiments, the dimensions of the channel are selected such that the biological fluid is drawn into the channel by capillary action.

Preferably, the metering stack 224 is designed to direct the biological fluid to flow into the channel 230 and into any receiving chamber(s) that may be present. In some embodiments, the channel 230 can be formed of or coated with a hydrophilic material, non-limiting examples of which include 93210 hydrophilic PET (Adhesives Research, Glen Rock PA) or 9984 Diagnostic Microfluidic Surfactant Free Fluid Transport Film, 9960 Diagnostic Microfluidic Hydrophilic Film, or 9962 Diagnostic Microfluidic Hydrophilic Film (3M Oakdale, MN).

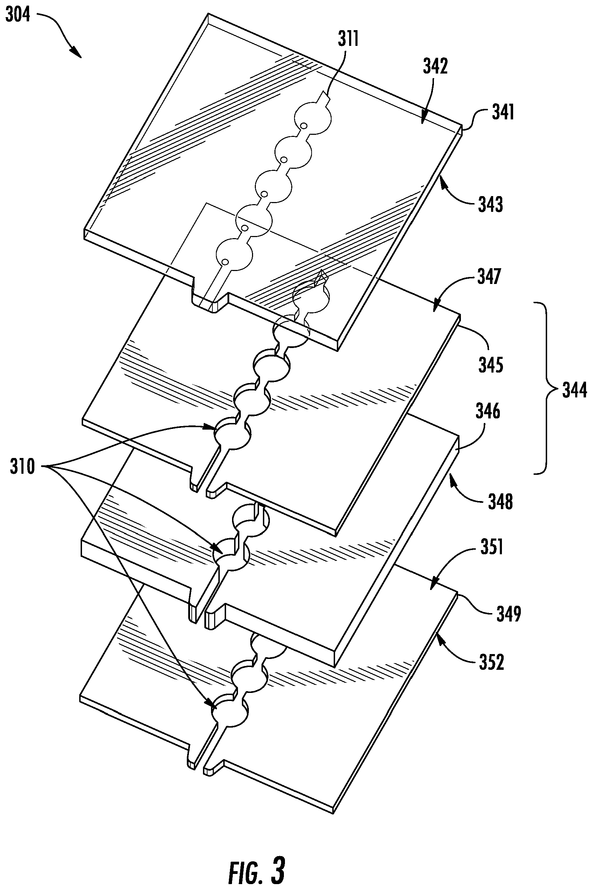

FIG. 3 illustrates an exploded view of a metering stack 304 according to one exemplary embodiment of the present disclosure, where such metering stack 304 can be used as metering stack 224 in the embodiment of FIGs. 2A to 2C. In FIG. 3, the metering stack 304 is formed by assembling multiple layers. The first layer 341 can be a plastic sheet with a first side 342, which is in communication with the surrounding environment when the cartridge is located outside the assay reader, and a second side 343 that faces the assay stack. In some embodiments, the first layer 341 may be a cover layer or top layer of the metering stack. In preferred embodiments, first layer 341 may have a hydrophilic surface or coating on second side 343. Non-limiting examples of suitable hydrophilic surfaces coatings include polyvinylpyrrolidone-polyurethane interpolymer, poly(meth)acrylamide, maleic anhydride polymers, cellulosic polymers, polyethylene oxide polymers, and water-soluble nylons or derivatives thereof, to name just a few. The presence of the hydrophilic surface or coating on second side 343 helps to draw the biological fluid (e.g., blood fluid sample) into the channel. The first layer 341 may include venting holes 311 positioned to align with the channel 310 defined by the layers below. In FIG. 3, for example, the venting holes 311 are aligned with the receiving chambers of channel 310 to allow air that otherwise would be trapped as an air bubble in the receiving chamber during channel filling to escape efficiently into the surrounding environment. It should be noted that the channel opening can also serve as a vent hole, if desired. In certain preferred embodiments, the first layer 341 includes polyethylene terephthalate (PET) with a hydrophilic coating on the second side 343 and venting holes 311.

The second layer 344 is positioned below the first layer 341 on the second side or assay facing side of the first layer 341. The second layer 344 itself can be a combination of one or more layers as illustrated in FIG. 3. Regardless of whether the second layer is comprised of one layer or more than one layer, the second layer essentially defines the shape and size of channel in the metering stack, including any receiving chambers that may be part of the channel. For example, the second layer 344 can be formed from one or more layers of polymeric material cut to define the volume and shape of the channel 310 that can contain the biological fluid (e.g., blood fluid sample). Other non-limiting methods of forming the channel 310 include injection-molding, stamping, machining, casting, laminating, and 3-D printing. Combinations of such fabrication techniques are also expressly contemplated by the present disclosure. In the embodiment shown in FIG. 3, second layer 344 has a first side 347 facing the first layer 341 and an opposite, second side 348 that faces the assay stack. Furthermore, second layer 344 includes adhesive layer 345 and plastic layer 346. Adhesive layer 345 fastens the first layer 341 to plastic layer 346. In some embodiments, the second layer 344 can be a combination of one or more plastic layer(s) 346 and adhesive layers 345. Preferably, adhesive layer 345 or plastic layer 346 or both are fabricated from materials which present a hydrophilic surface to the interior surfaces of the channel 310 in order to facilitate the distribution of the biological fluid within channel 310. In some embodiments, the hydrophilic plastic sheet(s) can include a PET material with a channel 310 cut into it. If desired, channel 310 may include one or more receiving chambers as shown in FIG. 3. Thus, the thickness and geometry of channel 310 can control the volume of sample to be collected. The hydrophilic interior surfaces of the channel 310 allow the metering stack to collect blood sample by capillary force. In some embodiments, the first layer 341 and the second layer 344 can be one integrated layer used in the metering stack 304.

In FIG. 3, third layer 349 can be formed from a hydrophobic adhesive layer. Non-limiting examples of suitable materials for fabricating third layer 349 include 3M 200MP adhesive or 3M 300MP adhesive (3M, Oakdale, MN). In preferred embodiments, the same channel geometry as channel 310 is cut into the third layer to match channel 310 cut in the second layer. In some embodiments, the third layer 349 can have a first side 351 facing the second layer 344 and a second side 352.

The method used to fabricate the metering stack is not particularly limited, so long as it is compatible with the general manufacturing requirements for medical devices. In certain embodiments, the layers that constitute the metering stack are first fastened together as large multilayer sheet or strip which is then subjected to stamping or cutting processes to form the metering stack, including the channel and any receiving chambers that may be present. In some embodiments, the first layer 341 and second layer 344 can be combined in one piece of plastic material with a hydrophilic surface forming the channel. Various other combinations of two or more layers, as well as additional layers, are contemplated by various embodiments.

In the assay device or POC systems of the present disclosure, the assay reactions occur in the assay stack of the assay device. In general, an assay stack includes one or more "assay components." As used herein, the term "assay component" refers to one or more of the active component and a passive supporting element or mask, including but not limited to the multiplexed assay pads. The number of assay pads (e.g., separation membranes, detection membranes, etc.) in a particular assay component is not particularly limited but is based on the particular assay requirements needed to diagnose the condition or analyze the fluid sample of the patients for whom the assay stack is designed. In preferred embodiments, the layers of the assay pads of a given assay component align vertically with the appropriate regions of the channel in the metering stack above to ensure that a predetermined volume of a biological fluid, sufficient to perform the assay associated with the particular target analyte of interest, is delivered to the detection membrane. The assay pads can act as a wick that draws the sample through the metering stack into the assay stack, for example through capillary action, gravity, etc. Therefore, once the metering stack and the assay stack are in contact with or within close proximity to each other, the biological fluid to be analyzed is directed to move into the detection membrane, where it may encounter one or more reagents required to perform the assay associated with the particular assay component. If desired, the assay stack may comprise additional layers that contain reagents required for the completion of the assay. The number of layers required can depend on the number of chemical reactions that need to take place in order to complete the assay. In various embodiments, layers of the assay stack can be made of variously shaped and variously-sized pads of different porous membrane materials, non-limiting examples of which include a sulfone polymer (e.g., a polysulfone, a polyethersulfone, a polyarylsulfone, etc.), nylon, cellulose (e.g., nitrocellulose, cellulose filter paper, etc.) and glass fiber.

The type of assays that may be performed using the assay systems of the present disclosure are not particularly limited and can be any assay for which the required reagents can be stably incorporated into one or more separation membranes and/or detection membranes and which can cause a change that can be detected by the assay reader. In some embodiments, the assay reactions cause a color change, which may be detected using the colorimetric detection methods as described herein. Still other assay reactions may result in another optical change, a fluorescence change, an electrochemical change, or any other detectable change that may occur in a detection membrane of the assay stack. In certain embodiments, the assays may be porous material-based lateral flow assays, vertical flow assays, and/or a combination of lateral and vertical flow assays. In general, the target analyte is contained within a biological fluid, non-limiting examples of which include blood, plasma, serum, saliva, sweat, urine, lymph, tears, synovial fluid, breast milk, and bile, or a component thereof, to name just a few. In certain preferred embodiments, the biological fluid is blood. For example, in one embodiment, the assay systems of the present disclosure are useful for providing patients with POC information regarding target analytes in their blood composition. In particular, the assay systems of the present disclosure can be used to determine the concentration of HDL, LDL, VLDL, total cholesterol, or combination thereof in a blood fluid sample. Other analytes that can be measured in blood via other receiving chambers that may be present as part of the assay system include thyroid markers (e.g., T3, free T4, thyroid stimulating hormone, etc.), inflammatory markers (e.g., C-reactive protein, etc.), vitamins (detected via a competitive assay structure), metabolic syndrome markers, glucose, glycated hemoglobin, glycated albumin, and serological levels of antibodies against a disease (detected by a labeled antigen architecture). Non-limiting examples of analyte that can be measured in urine include total protein, leukocyte esterase and myoglobin.

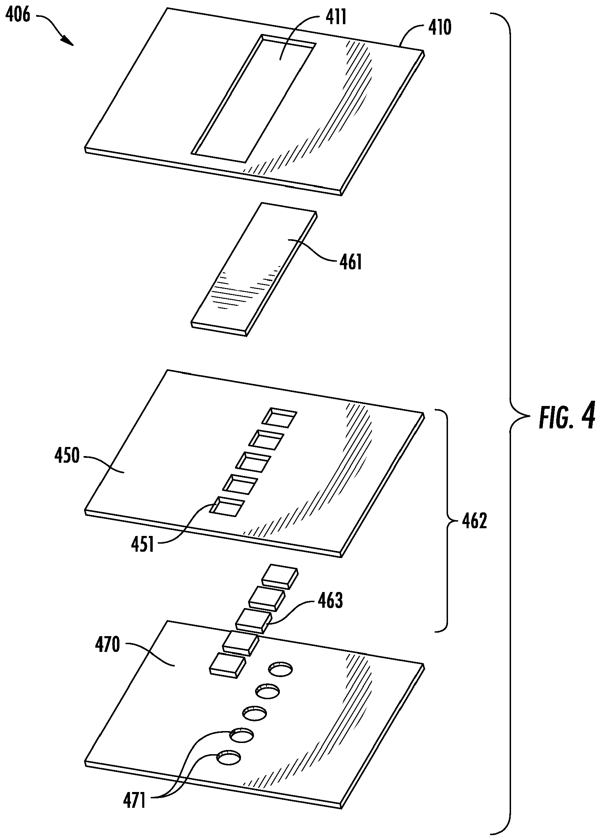

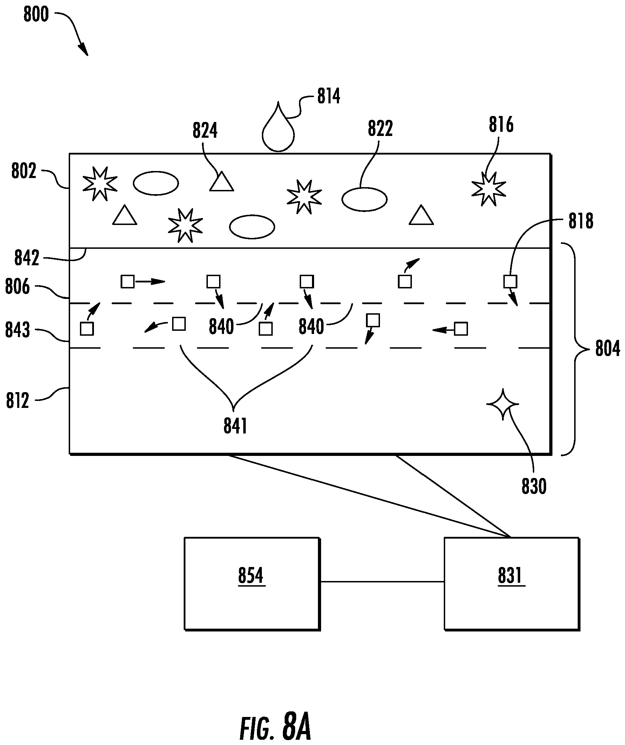

FIG. 4 illustrates an exemplary assay stack 406 of an assay device according to one embodiment of the present disclosure, where such assay stack 406 can in particular be used as assay stack 226 in the embodiment of FIGs. 2A to 2C. In FIG. 4, the assay stack 406 is formed of multiple layers, including one or more of the layers with active components and a passive supporting element or mask. More specifically, in FIG. 4, assay stack 406 includes assay stack cover layer 410 that features a cut-out portion 411 that is aligned with the channel in the overlying metering stack. Generally, assay stack cover layer 410 is fabricated from a polymeric material that provides rigidity to the assay stack and provides ease of handling during manufacturing of the cartridge. Furthermore, the cut-out portion 411 allows the biological fluid to flow past the assay stack cover layer 410 towards the underlying assay components when the cartridge is inserted into the assay reader, as described herein. As shown, the assay stack 406 includes a separation membrane 461 which can be the top-most layer facing the metering stack. The separation membrane 461 is used to separate components of the biological fluid (e.g., blood fluid sample) to prevent undesirable components from reaching the underlying assay components. For example, when the biological fluid is a blood fluid sample and the assay is focused on determining an HDL concentration in the blood fluid sample, the separation membrane 461 is designed specifically to prevent red blood cells and non-HDL proteins (e.g., LDL, VLDL, etc.) from reaching the detection membrane portion of the assay stack 406 after the cartridge has been inserted into the assay reader. This is advantageous because the strong spectral absorption by the hemoglobin present in red blood cells may overwhelm the color changes that occur in the detection membrane 463 of the assay stack 406 during performance of the assay. Further, the presence of non-HDL proteins in the detection membrane 463 of the assay stack 406 may also lead to interference and reduced accuracy in determining the HDL concentration in the blood fluid sample, so the separation membrane 461 is specifically designed to trap such non-HDL proteins and prevent their passage to the detection membrane 463. Such a separation membrane 461 can be made of a variety of materials, non-limiting examples of which include a sulfone polymer (e.g., a polysulfone, a polyethersulfone, a polyarylsulfone, etc.), nylon, cellulose (e.g., nitrocellulose, cellulose filter paper, etc.) and glass fiber. In some embodiments, the fabrication of the separation membrane can include surface treatments for improved wettability and/or other properties. The separation membrane can be one continuous piece of membrane for all of the assay components, or multiple discontinuous pieces of membrane material that may be the same or different (or some combination thereof) for each of the detection membranes in the assay component in the assay stack FIG. 4. When the separation membrane 461 is discontinuous, cross-talk between neighboring assays can be prevented. In some embodiments, some of the detection membranes of an assay component have a corresponding separation membrane, while other detection membranes do not have such a layer. Other additional components and features of the separation membranes of the assay devices contemplated by the present disclosure are discussed in more detail with respect to FIGs. 8A-8D.

As shown in FIG. 4, in some embodiments, the assay stack 406 can include an assay component 462 positioned below the separation membrane 461. The assay component 462 includes a mask support layer 450 with a plurality of cut-outs 451 that are configured to receive and immobilize detection membranes 463 when the assay stack 406 is assembled. Preferably, cut-outs 451 are positioned to align detection membranes 463 with separation membranes 461 such that the biological fluid containing the target analyte will flow from separation membranes 461 into detection membranes 463. Detection membranes 463 (e.g., detection membranes such as but not limited to color generation membranes) may comprise reagents that are necessary to complete the assay reactions that are initiated once the target analyte (e.g., HDL or other protein of interest) flows through the separation membranes 461. In some embodiments, detection membranes 463 serve as a detection indicator layer that provides information corresponding to the results of the assay performed. For example, detection membranes 463 (e.g., color generation membranes) can include a visual indicator, such as a color change, to indicate the results of the assays, although it is to be understood that the detection membranes contemplated by the present disclosure also contemplate fluorescent and electrochemical changes or responses. Furthermore, while assay stack 406 in FIG. 4 contains only assay component 462, it should be understood that the assay stack 406 may contain additional assay components with assay pads that are impregnated with the reagents required to complete and/or report the results of a particular assay. For instance, the assay stack 406 can include any number of assay components necessary to perform the analysis of the blood sample. Because some assays require more chemical steps than others, assay components may comprise more non-functional assay pads which only serve to draw the completed assay products to the bottom of the assay stack, where the results may be detected by the assay reader, as described herein.

Assay stack 406 in FIG. 4 also includes an assay bottom layer 470, which is typically fabricated from a polymeric material to provide mechanical strength and ease of handling of assay stack 406 during the manufacturing process. In addition, assay bottom layer 470 typically includes a plurality of detection ports 471 which are aligned with the detection membranes of the assay stack and sized to permit interrogation of the assay results by the assay reader.

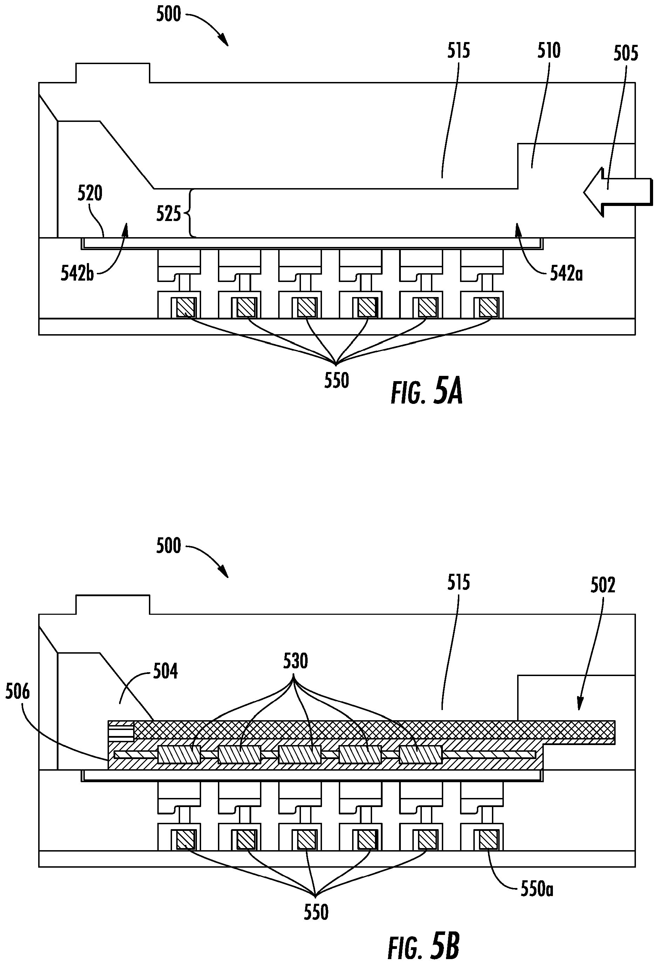

FIG. 5A shows a schematic drawing of an assay reader, in longitudinal cross-section, according to one non-limiting embodiment of the present disclosure. In FIG. 5A, assay reader 500 includes cartridge receiving chamber 510 which houses the cartridge when it is inserted as indicated by arrow 505. Tab 515 runs longitudinally along assay reader 500 and extends into cartridge receiving chamber 510. Tab 515 is configured to insert into a slot at the top of the cartridge, such as slot 228 in FIG. 2C, when the cartridge is inserted into the assay reader. In addition, the spacing 525 between the bottom edge of tab 515 and support surface 520 is set such that when the cartridge is inserted, tab 515 compresses the metering stack and the assay stack together, thereby causing the biological fluid containing the target analyte to flow from the metering stack into the assay stack and initiating the assay reactions. In certain embodiments, the assay reader may comprise a snap-fit mechanism that locks the cartridge in place once it has been fully inserted into the assay reader. This is advantageous because it prevents the user from accidentally removing the cartridge from the assay reader before the assays are complete, which could adversely affect the accuracy of the assay results. In some embodiments, assay reader 500 also includes sensors 542a and 542b, which detect and time the insertion of the cartridge. For example, as the cartridge is inserted into cartridge receiving chamber 510 and begins to engage with tab 515, the bottom surface of the cartridge may pass over sensor 542a, which is detected by appropriate electronics as the beginning of the insertion of the cartridge. The second sensor 542b is located further inside the assay reader 500 and detects the presence of the cartridge when the cartridge is fully inserted as well as the time at which full insertion occurred. Assay reader 500 may then compare the overall time for insertion of the cartridge to determine if the insertion of the cartridge was timely and proper. In this way, the assay reader will not perform any assay readings in situations where (1) the cartridge was only partially inserted, or (2) the cartridge was partially inserted, removed, and inserted again. Either case could give inaccurate assay readings, due to the incomplete compression of the metering stack and assay stack, resulting in incomplete delivery of the required amount of target analyte to the detection membranes in the assay stack.

In the exemplary embodiment shown in FIG. 5A, assay reader 500 detects the results of the assay by detecting the color change of the detection membrane caused by the assay reactions. To achieve this, assay reader 500 includes a plurality of light sources (not shown in this cross-sectional drawing) and light detection elements 550 arrayed within assay reader 500 such that they align with the detection membranes of the cartridge when the cartridge is fully inserted. In order for light detection elements 550 to be able to detect the color of the detection membranes, support surface 520 may be equipped with one or more apertures or be fabricated from a transparent material that allows light to penetrate therethrough. However, it is also to be understood that the assay reader 500 can alternatively include components to detect electrochemical or fluorescent changes in a detection membrane portion of the assay stack. FIG. 5B shows a schematic illustration of a longitudinal cross-section of assay reader 500 with cartridge 502 fully inserted. Cartridge 502, which may correspond to cartridge 100 or 200 of FIG. 1 or FIGs. 2A to 2C, includes metering stack 504 and assay stack 506, which are compressed together by tab 515 such that the target analyte (e.g., HDL) is delivered from the metering stack 504 to the detection membranes 530. Detection membranes 530 are aligned with light detection elements 550. Note, however, that assay reader 500 may include an additional light detection element 550a without a corresponding detection membrane 530. The presence of additional light detection elements, such as light detection element 550a, allow the assay reader to be used with different types of cartridges for different assays, particularly cartridges that may be designed to perform more assays, as well as to identify the different types of cartridges for the different assays.

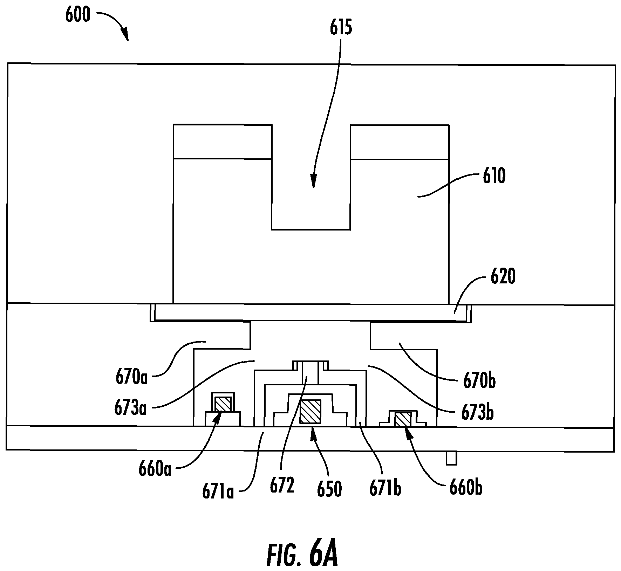

FIG. 6A shows a schematic drawing of a transverse cross-section of the assay reader shown in FIG. 5 in the form of an assay reader 600 that may be used to detect color changes. In FIG. 6A, the assay reader 600 includes a tab 615 that extends into cartridge receiving chamber 610 to engage with a slot on the cartridge. Such engagement then compresses the metering stack and the assay stack against support surface 620, initiating the assay reactions. Light sources 660a and 660b provide light for detecting the assay results and are positioned near light detection device 650. Specifically, as illustrated in FIG. 6A, light sources 660a and 660b provide light to analyze the detection membrane corresponding to light detection device 650. In general, it is advantageous to dedicate one or more light sources to each light detection element in order to ensure that the photon flux onto the light detection element is sufficient to obtain an accurate reading. In some embodiments, the light sources dedicated to a particular light detection element have the same output spectrum. In other embodiments, however, the light sources corresponding to a given light detection element produce different output spectra. For instance, the light sources may be light emitting diodes (LEDs) that produce different colors of light. In general, it is advantageous to include optical elements to direct the light and/or reduce the amount of light scattering in the assay reader. In some embodiments, the optical elements are apertures that only allow light emanating from the light source that is line-of-sight to the respective detection membrane to reach the detection membrane. For example, in FIG. 6A, light source 660a is limited by aperture defining members 670a and 671a such that only the light from light source 660a that passes through aperture 673a will reach the detection membrane and subsequently be detected by light detection device 650. Similarly, light source 660b is limited by aperture defining members 670b and 671b, such that only the light from light source 660b that passes through aperture 673b will reach the detection membrane and subsequently be detected by light detection device 650. In preferred embodiments, aperture defining members 670a, 670b, 671a, and 671b are fabricated from a black matte material to reduce the amount of undesirable scattering when light sources 660a and 660b are turned on. Furthermore, in this embodiment, light detection device 650 located in a housing that is comprised of aperture defining members 671a and 671b that only permit light that passes through aperture 672 to reach light detection device 650. If desired, the aperture 672 may be fitted with a filter to admit only light of a predetermined wavelength or wavelength range for detection by light detection device 650. This may be useful, for example, when the light sources are equipped to provide only white light for colorimetric analysis. In addition, the light from light sources 660a and 660b and the light to be detected by light detection device 650 may be directed or manipulated using optical elements such as lenses, filters, shutters, fiber optics, light guides, and the like without departing from the spirit and the scope of the present disclosure.

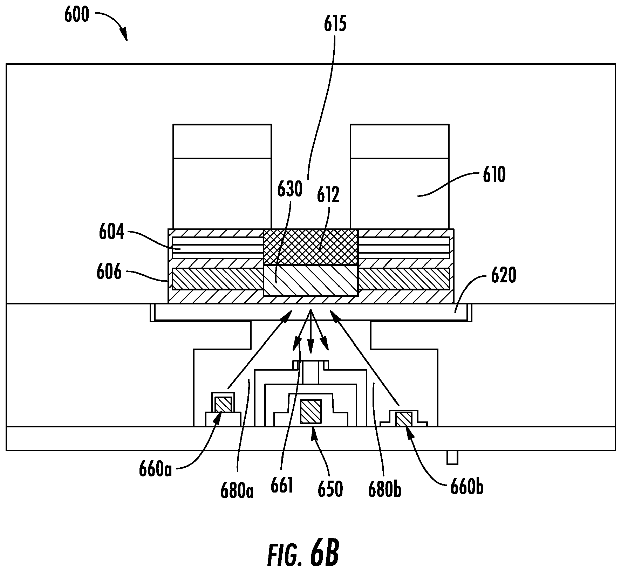

FIG. 6B shows a schematic illustration of the operation of the assay reader described in FIG. 6A. In FIG. 6B, a cartridge comprising metering stack 604 and assay stack 606 are inserted into cartridge receiving chamber 610 of assay reader 600. Tab 615 compresses metering stack 604 and assay stack 606 against support surface 620 to cause the fluid sample (e.g., blood fluid sample) to flow from the channel 612 into detection membrane 630. As noted previously, assay reader 600 may be fitted with sensors to confirm that the cartridge has been inserted correctly and in a timely manner. Assay reader 600 may also be pre-programmed before sample collection, either by the user or during the manufacturing process, to illuminate the detection membranes at the appropriate time based on the type of cartridge being used. In this way, assay reader 600 collects assay data from detection membrane 630 only when the assay is completed. Alternatively, if desired, assay reader 600 may be configured to collect assay data from detection membrane 630 during the entire assay reaction after the cartridge has been inserted. As shown in FIG. 6B, light source 660a provides light beam 680a, which impinges on the bottom face of detection membrane 630 to produce reflected light beam 661. Similarly, light source 660b produces light beam 680b, which may impinge on the bottom of the detection membrane 630 to produce reflected light beam 661 at the same time as light source 660a or a different time, depending on the requirements of the assays being detected.

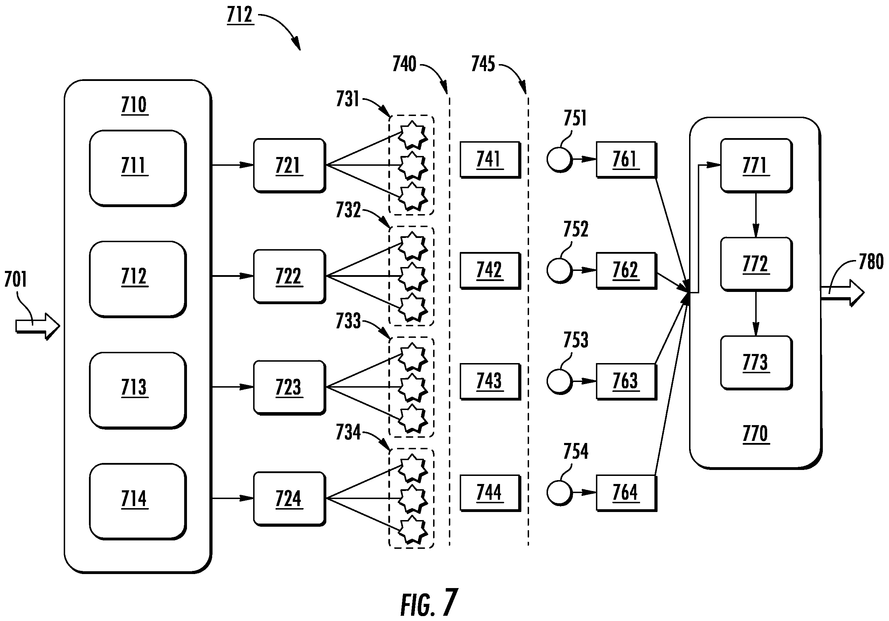

FIG. 7 shows a block diagram 700 of a sensor configuration inside an assay reader according to one exemplary embodiment of the present disclosure. In FIG. 7, four detection membranes (identified by reference numerals 741, 742, 743, and 744) have completed their assay reactions with the target analyte, undergone the respective color changes, and are ready for colorimetric analysis. Note that, if desired, this configuration can also be used to collect data from the four detection membranes to monitor the progress of the assay reactions. Input signal 701 from a first microcontroller serial-peripheral interface bus (MCU SPI Bus) enters digital-to-analog converter unit 710, which includes individual digital-to-analog converters 711, 712, 713, and 714 that independently control current sources 721, 722, 723, and 724. These current sources, in turn, power light sources 731, 732, 733, and 734, respectively. In some embodiments, input signal 701 may be sent by a timing circuit at a predetermined time after the insertion of the cartridge into the assay reader. In such embodiments, the predetermined time corresponds to the known time or times for the assay reactions in the detection membranes to reach completion. In some preferred embodiments, the light sources 731, 732, 733, and 734 are activated at the same time to measure the assay-induced color change of detection membranes 741, 742, 743, and 744 simultaneously in a multiplexed mode. However, this present disclosure also contemplates operating all of the light sources separately and sequentially, or some simultaneously and some separately, depending on the timing requirements of the assays in the cartridge.

In this non-limiting example, each of light sources 731, 732, 733, and 734 includes individual three light emitting diodes (LEDs) which may be the same or different colors, depending on the requirements of the assay and any optical elements that may be present in the assay reader. For example, in certain embodiments, the three LEDs in a particular light source (e.g., 731) may be red, green, and blue (RGB LEDs), such that the light impinging on the detection membrane is white light when all three LEDS are activated. Of course, the light sources are not limited to any particular number or type of LEDs or other light generating devices. More generally, the light sources that are useful in the assay readers of the present disclosure are not particularly limited, so long as they provide light of suitable wavelength(s) and brightness for the light detection element to make an accurate reading of the colored light reflected from the detection membrane. In certain non-limiting embodiments, the light sources are light emitting diodes (LEDs), organic light emitting diodes (OLEDs), active matrix organic light emitting diodes (AMOLEDs), or lasers. For example, the light source may be only one LED that has sufficient brightness and the proper wavelength to allow colorimetric analysis of an assay reaction in a given detection membrane. In certain embodiments, the light sources may produce light of specific wavelengths. Alternatively, the light source may be a broadband source that is paired with one or more narrow bandpass filters to select light of certain desired wavelength(s). Typically, the light sources produce light in the visible region of the electromagnetic spectrum (i.e., wavelength between 400 - 700 nm) although this present disclosure also contemplates light sources that produce electromagnetic radiation in the infrared (700 nm to 10⁶ nm) or ultraviolet regions (10 nm - 400 nm) of the electromagnetic spectrum, so long as they are paired with the appropriate light detection devices. Combinations of different light sources are also expressly contemplated by the present disclosure.

In FIG. 7, element 740 is a schematic representation of optical elements that optionally may be present in the optical path between the light sources 731, 732, 733, and 734 and detection membranes 741, 742, 743, and 744. When desired, one or more optical elements may be located between the light source and its corresponding detection membrane to direct the light, focus the light, reduce undesirable scattering, select one or more wavelengths for assay detection, or some combination thereof. Non-limiting examples of such optical elements include apertures, lenses, light guides, bandpass filters, optical fibers, shutters, and the like. Similarly, element 745 represent optical elements that optionally may be present in the optical path between detection membranes 741, 742, 743, and 744 and corresponding light detection devices 751, 752, 753, and 754. These optical elements may be used to manipulate the light upstream of the light detector devices in a manner similar to that described for element 740. It is to be understood that different types and numbers of optical elements may be used for each combination of light source, detection membrane, and light detection device. Light detecting devices 751, 752, 753, and 754 detect the light from the detection membranes 741, 742, 743, and 744. In this non-limiting example, the light detecting devices are photodiodes. More generally, the type of light detection device is not particularly limited, provided that it is capable of detecting the light that is reflected from the detection membranes used for colorimetric measurement of the assay results. Other examples of suitable light detection elements include photodiode arrays, CCD chips, and CMOS chips. The outputs from light detection devices (e.g., photodiodes) 751, 752, 753, and 754 are sent to transimpedance amplifier/low pass filter elements 761, 762, 763, and 764, which convert the current signal from the photodiodes to a voltage output, while filtering unwanted signal components. The output from transimpedance amplifier/low pass filter elements 761, 762, 763, and 764 are sent to analog-to-digital converter unit 770, which includes multiplexer unit 771, gain 772, and analog-to-digital converter 773. The output of analog-to-digital converter unit 770 may be sent to a component 780, which may be a second MCU SPI bus, a transmitter, or a processor. In certain embodiments, the transmitter allows for hardwired or wireless connectivity (e.g., Bluetooth or Wi-Fi) with a personal computer, mobile device, or computer network. In one particularly useful embodiment, the assay results are transmitted to the user's mobile device or personal computer, where they are displayed in a graphical user interface (GUI). If desired, the GUI may display prior assay results, in addition to the current results, in order to provide the user with information regarding the overall trends in the results of the assays. In addition, the assay results may be transmitted from the user's mobile device or computer to a computer network, such as one belonging to the user's physician. In this way, the assay systems of the present disclosure can allow a user's physician to monitor a patient closely, by providing up-to-date medical information from the assay results obtained by the assay reader.

It should be noted that the optical detection systems described in the foregoing correspond to some exemplary embodiments of the system, but that the present disclosure expressly contemplates other types of detection systems as well. In general, any detection system which corresponds to a signal change caused by an assay reaction may be used in connection with the assay reader of the present disclosure. Thus, for example, in certain embodiments, the detection system is an optical detection system that is based on chemiluminescence. In such embodiments, light sources such as LEDS and OLEDS are not required to detect a color change caused by the assay reaction in the detection membranes. Rather, the signal change may be caused by the reaction of an oxidative enzyme, such as luciferase, with a substrate which results in light being generated by a bioluminescent reaction. In another exemplary embodiment, the signal change caused by the assay reaction may be detected by electrochemical reaction.

FIGs. 8A-8D illustrate a further embodiment of cartridges 100, 200, or 502 in the form of a cartridge 800 that includes a metering stack 802 and an assay stack 804 during various stages of performing an assay after a blood fluid sample 814 containing a first protein 824, a second protein 816, and red blood cells 822, inter alia, has been introduced to the cartridge 800. The metering stack 802 is configured to receive and distribute the blood fluid sample 814 along a channel, where the channel has one or more venting holes in communication with the channel, as described in detail above. As also described above, a spacer material may be disposed between the metering stack and the assay stack, wherein the spacer material provides a gap between the metering stack and the assay stack that prevents the target analyte from flowing from the metering stack into the assay stack when the cartridge is in an uncompressed state. Additionally, the blood fluid sample 814 can flow from the metering stack 802 to the assay stack 804 upon compression of the cartridge 800.

Once introduced to the metering stack 802, the blood fluid sample 814 passes to the separation membrane 806, and ultimately, the target analyte reaches a detection membrane 812 (e.g., a color generation membrane) of the assay stack 804 as discussed in more detail below. It should be understood the separation membrane 806 can be referred to as the plasma separation membrane, where the separation membrane 806 allows for the separation of red blood cells 822 and a first protein (e.g., non-HDL) 824 from the blood fluid sample while allowing for a target analyte such as second protein 816 (e.g., HDL) to pass through the separation membrane 806 to the detection membrane 812. Further, the separation membrane 806 can include pores that have a pore size large enough to allow the second protein 816 to pass through to the detection membrane 812 but that also have a pore size small enough to prevent the passage of any red blood cells and the first protein 824 (that is bound to a first reagent (e.g., precipitating reagent) 818 specific for the first protein 824 present in the separation membrane 806, as discussed in more detail below, to the detection membrane 812, which could affect the accuracy of the assay results. This is because there is a strong spectral absorption by the hemoglobin present in red blood cells that may, for example, overwhelm the color changes that occur after the assay is performed. Further, the presence of the first protein 824 in the detection membrane 812 would also affect the accuracy of the detection of the amount of the second protein 816 via the detection membrane 812.

In one particular embodiment, the separation membrane 806 can include a first plurality of pores 840 located towards the upstream side 842 of the separation membrane 806 and a second plurality of pores 841 located towards the downstream side 843 of the separation membrane 806. The first plurality of pores 840 have an average pore size that is larger than an average pore size of the second plurality of pores 841. For instance, the first plurality of pores 840 in the separation membrane 806 can have an average pore size ranging from about 10 micrometers to about 150 micrometers, such as from about 15 micrometers to about 125 micrometers, such as from about 20 micrometers to about 100 micrometers, while the second plurality of pores 841 in the separation membrane 806 can have an average pore size ranging from about 0.1 micrometers to about 7.5 micrometers, such as from about 0.5 micrometers to about 5 micrometers, such as from about 1 micrometer to about 2.5 micrometers. A separation membrane 806 having such a pore size arrangement and distribution can be referred to as an asymmetric membrane, and the ratio of the average pore size of the first plurality of pores 840 to the average pore size of the second plurality of pores 841 can range from about 1.3 to about 1500. Thus, the separation membrane 806 has pore sizes that are large enough to allow the second protein 816 to pass through to the detection membrane 812 but that also have a pore size small enough to prevent the passage of any red blood cells 824 and first protein complex 824 to the detection membrane 812, which could affect the accuracy of the assay results.

Alternatively, or in addition to the pore size of the separation membrane 806 being utilized to prevent the passage of the red blood cells 822 and the first protein complex 828 to the detection membrane 812, the separation membrane 806 may also have a structure that defines a tortuous path. The tortuous path makes it difficult and significantly slows down the ability of large, sticky components like red blood cells 822 and a complex 828 formed by the first protein 824 bound to the first reagent (e.g., precipitating reagent) 818 to pass through to the detection membrane 812. Meanwhile, the smaller, unbound second protein 816 is able to pass quickly through the tortuous path of the separation membrane 806 and to the detection membrane 812, where an accurate reading of the amount of second protein 816 in the blood fluid sample 814 can be determined before any lingering red blood cells 822 and complexes 828 are able to pass through to the detection membrane 812.

Additionally, as shown in FIG. 8A and as mentioned above, the separation membrane 806 includes a first reagent (e.g., precipitating reagent) 818 that is specific for the first protein (e.g., non-HDL) 824. It should be understood that for ease of manufacturing and for efficiency purposes, the precipitating reagent 818 is not immobilized on the separation membrane 806. In some embodiments, the precipitating reagent 818 includes a polyanionic non-high density lipoprotein binding reagent. For instance, the precipitating reagent 818 can include a sulfonated polysaccharide, heparin, or phosphotungstic acid. In addition, the precipitating reagent 818 can also include a group II cation. The weight ratio of the sulfonated polysaccharide, heparin, or phosphotungstic acid to the group II cation can range from about 9:1 to about 1:2, such as from about 8:2 to about 1:1.5, such as from about 7:3. In some embodiments, the group II cation can include Mg, Mn, or Ca. In one particular embodiment, the precipitating reagent 818 can include phosphotungstic acid and Mg. For example, the precipitating reagent 818 can include phosphotungstic acid and magnesium chloride hexahydrate (MgCl₂). In other embodiments, the precipitating reagent 818 can include heparin and manganese (II) chloride, dextran sulfate, or magnesium chloride.

Further, the precipitating reagent 818 can be present in separation membrane 806 at a concentration ranging from about 35 milligrams/milliliter to about 250 milligrams/milliliter, such as from about 45 milligrams/milliliter to about 245 milligrams/milliliter, such as from about 55 milligrams/milliliter to about 240 milligrams/milliliter based on the wet weight of the precipitating reagent 818 present on the detection membrane 812. In addition, because about 20 microliters to about 40 microliters of the precipitating reagent 818 can be applied to the separation membrane per square centimeter of the separation membrane 806, the precipitating reagent 818 can be present in the separation membrane 806 at a concentration ranging from about 0.7 milligrams/square centimeter to about 10 milligrams/square centimeter, such as from about 0.9 milligrams/square centimeter to about 9.8 milligrams/square centimeter, such as from about 1.1 milligrams/square centimeter to about 9.6 milligrams/square centimeter based on the dry weight of the precipitating reagent 818. In still other embodiments, the precipitating reagent 818 can be present in the separation membrane at concentration ranging from about 1 milligrams/square centimeter to about 7.5 milligrams/square centimeter, such as from about 1.5 milligrams/square centimeter to about 7 milligrams/square centimeter, such as from about 2 milligrams/milliliter to about 6.5 milligrams/square centimeter. Additionally, the separation membrane 806 can have a surface area of about 0.25 square centimeters in some embodiments. In such embodiments, the precipitating reagent 818 can be present in the separation membrane 806 an amount ranging from about 175 micrograms to about 2500 micrograms, such as from about 225 micrograms to about 2450 micrograms, such as from about 275 micrograms to about 2400 micrograms. In still other embodiments, the precipitating reagent 818 can be present in the separation membrane 806 in an amount ranging from about 250 micrograms to about 1875 micrograms, such as from about 325 micrograms to about 1750 micrograms, such as from about 500 micrograms to about 1625 micrograms.

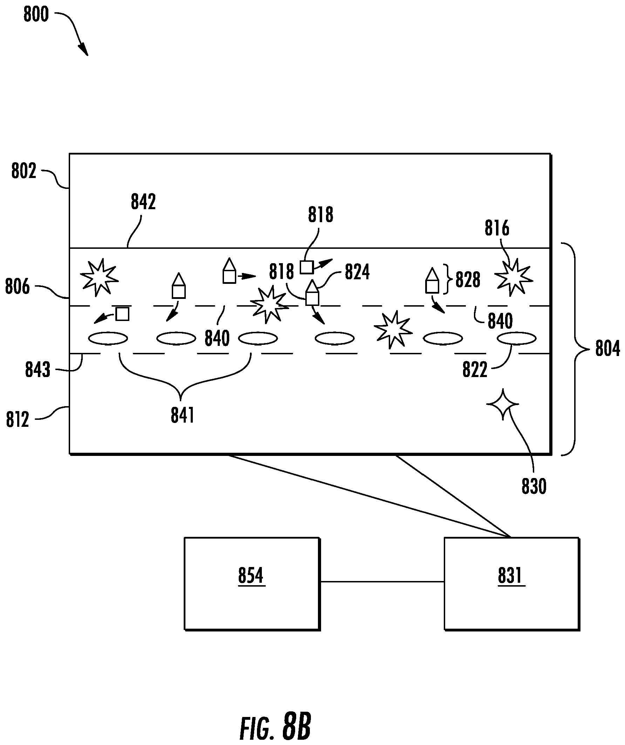

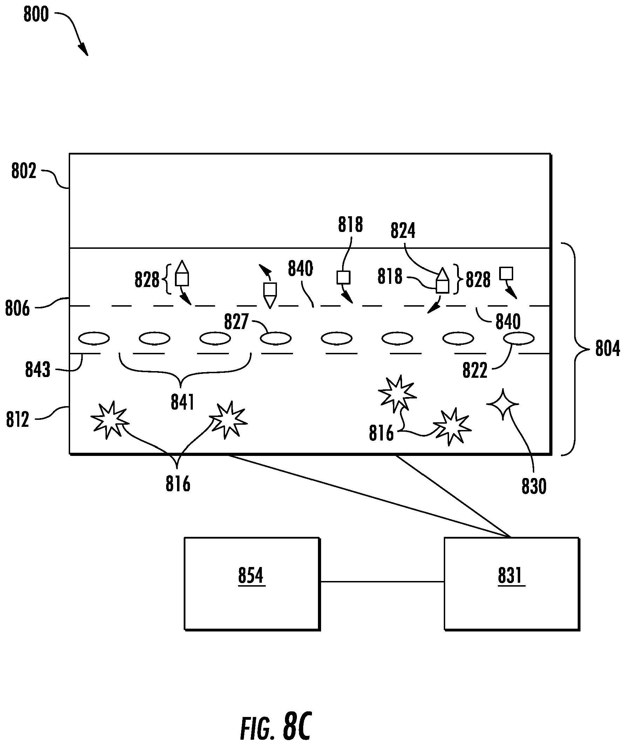

Ultimately, as shown in FIGs. 8B and 8C, any first protein 824 present in the blood fluid sample 814 can be precipitated by the precipitating reagent 818 present and mobilized in the separation membrane 806 to form a complex 828. As shown, the complex 828 and any red blood cells 822 present in the blood fluid sample 814 are trapped or slowed down by the pore size of the second plurality of pores 841 and/or the tortuous path structure of the separation membrane 806 such that only the second protein 816, which is unbound to any reagent, can quickly pass through to the detection membrane 812 for a determination of the amount of the second protein 816 present in the blood fluid sample 814 before any lingering red blood cells 822 and/or complexes 828 are able to reach the detection membrane 812. It should be understood that the separation of the red blood cells 822 and the first protein 824 thus occurs concurrently in the separation membrane 806 in a single layer of the assay stack 804, which improves the efficiency of such separation compared to conventional assays that separate red blood cells and non-HDL from HDL in a blood sample.

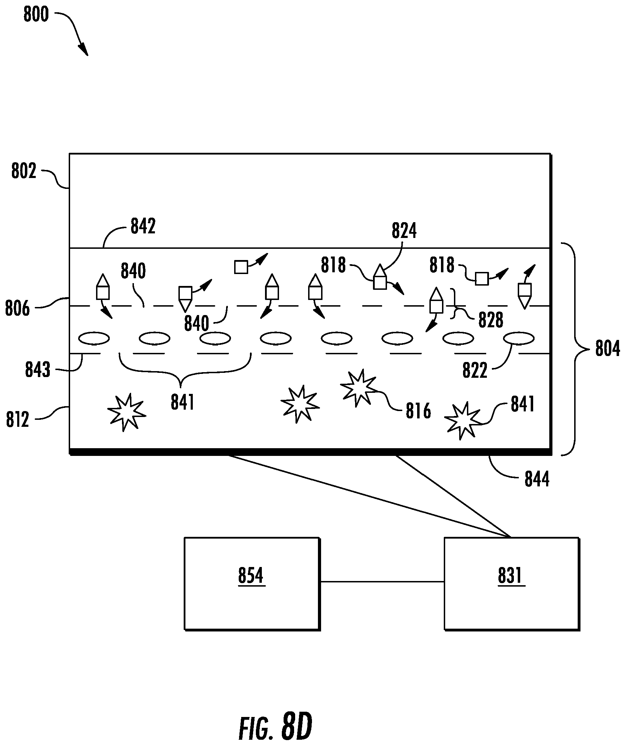

Turning now to FIGs. 8C-8D, after the second protein (e.g., HDL) 816 passes through the first plurality of pores 840 and the second plurality of pores 841 in the separation membrane 806, the second protein 816 can react with a second reagent 830 present in the detection membrane 812. The reaction between the second reagent 830 and the second protein 816 present can elicit a quantifiable response 844 (e.g., a colorimetric response, a fluorescent response, an electrochemical response, etc.) in the presence of the second protein 816, wherein the quantifiable response 844 corresponds to an amount of the second protein 816 present in the detection membrane 812 as shown in FIGs. 8C and 8D. In one embodiment, the second reagent 830 can be specific for HDL and can include cholesterol esterase, cholesterol oxidase, or a combination thereof, although any suitable reagents for measuring HDL are contemplated by the present disclosure and known to those having ordinary skill in the art. For example, in one embodiment, the second reagent 830 can include one or more reagents which are catalyzable by the second protein 816 to provide a detectable signal within the detection membrane 812.

In any event, the quantifiable response 844 (e.g., a colorimetric response, a fluorescent response, an electrochemical response, etc.) in the detection membrane 812 can be detected by one or more detection devices, which, for example, can include light detection devices 854 as shown in FIG. 8D when the quantifiable response is a color change. Meanwhile, one or more light sources 831 provide light for detecting the quantifiable response 844 (e.g., color change) in the detection membrane 812 (e.g., color generation membrane) and can be positioned near light detection device 864. In the case of a colorimetric response, the one or more light sources 831 provide light to analyze the quantifiable response 844 (e.g., color change) in the detection membrane 812 (e.g., color generation membrane) corresponding to light detection device 854. As described above, it is advantageous to dedicate one or more light sources to each light detection element in order to ensure that the photon flux onto the light detection element is sufficient to obtain an accurate reading. In some embodiments, the light sources 831 dedicated to a particular light detection device 854 have the same output spectrum. In other embodiments, however, the light sources 831 corresponding to a given light detection devices 854 produce different output spectra. For instance, the light sources may be light emitting diodes (LEDs) that produce different colors of light. In general, it is advantageous to include optical elements to direct the light and/or reduce the amount of light scattering in the assay reader. If desired, the light detection device 854 may be fitted with a filter to admit only light of a predetermined wavelength or wavelength range for detection by light detection device 854. This may be useful, for example, when the light sources 831 are equipped to provide only white light for colorimetric analysis. In addition, the light from light sources 831 and the light to be detected by light detection device 854 may be directed or manipulated using optical elements such as lenses, filters, shutters, fiber optics, light guides, and the like without departing from the spirit and the scope of the present disclosure. More generally, the light sources 831 that are useful in the assay readers of the present disclosure are not particularly limited, so long as they provide light of suitable wavelength(s) and brightness for the light detection devices 854 to make an accurate reading of the colored light reflected from the detection membrane 812.

It should also be understood that the detection membrane 812 can include one or more stabilizing agents (not shown). Such stabilizing agents can include neo silk protein saver; mannitol, trehalose, or other sugars; polypropylene glycol-polyethylene glycol block copolymers or other hydrophilic-hydrophobic block copolymers; or a combination thereof.

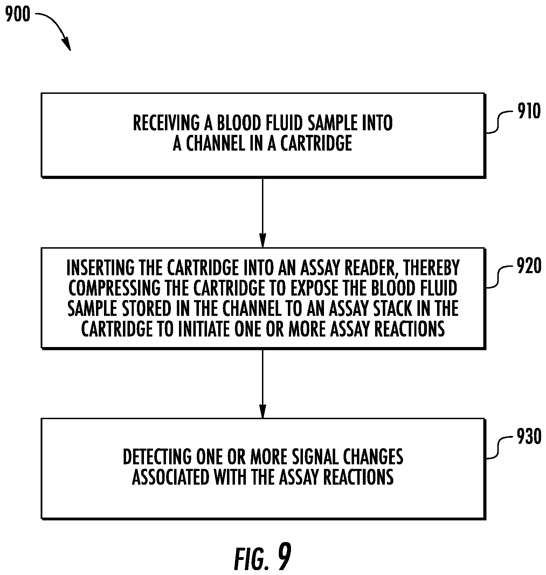

FIG. 9 shows a flowchart that illustrates a method 900 of using of the assay system according to one embodiment of the present disclosure to perform a plurality of assays. The method 900 includes step 910, which involves receiving a blood fluid sample that contains the target analyte (e.g., HDL) into a channel in a cartridge. Step 920 involves inserting the cartridge into an assay reader, thereby compressing the cartridge to the blood fluid sample stored in the channel to an assay stack in the cartridge to initiate one or more assay reactions. Step 930 involves detecting one or more signal changes associated with the plurality of assay reactions. The method 900 can include any additional steps that would be understood by one of ordinary skill in the art to detect the one or more signal changes via the various components of the metering stack and assay stack described in detail above.

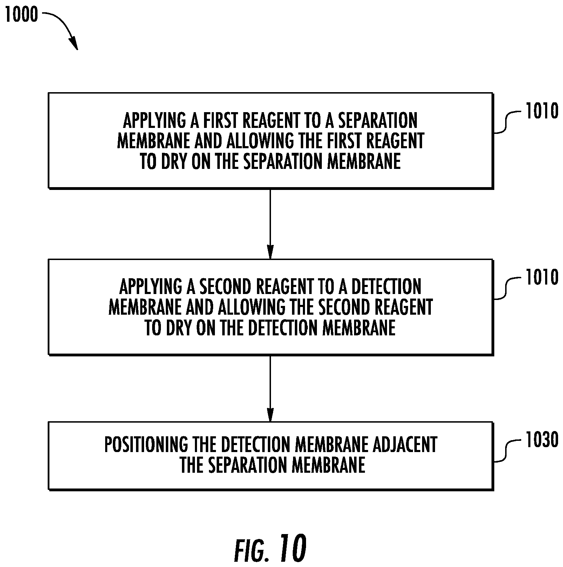

FIG. 10 shows a flowchart that illustrates a method 1000 of fabricating a cartridge according to one embodiment of the present disclosure. The method includes the steps of obtaining a separation membrane, applying a first reagent (e.g., a precipitating reagent for non-HDL) to the separation membrane, and allowing the first reagent to dry (step 1010). The method 1000 also includes applying a second reagent (e.g., a reagent specific to HDL) to a detection membrane and allowing the second reagent to dry on the detection membrane (step 1020). Further, the method 1000 also includes the step of positioning the detection membrane adjacent the separation membrane (step 1030). In utilizing an assay device fabricated by this method, the second reagent interacts with the second protein (e.g., HDL) in the detection membrane without interference from the first protein (e.g., non-HDL) or red blood cells to elicit a quantifiable response in when a blood fluid sample is introduced to the assay device. Further, the quantifiable response corresponds to an amount of the second protein present in the detection membrane, which corresponds to an amount or concentration of the second protein present in the fluid sample.

### EXAMPLES

The present disclosure may be better understood by reference to the following examples.

### EXAMPLE 1

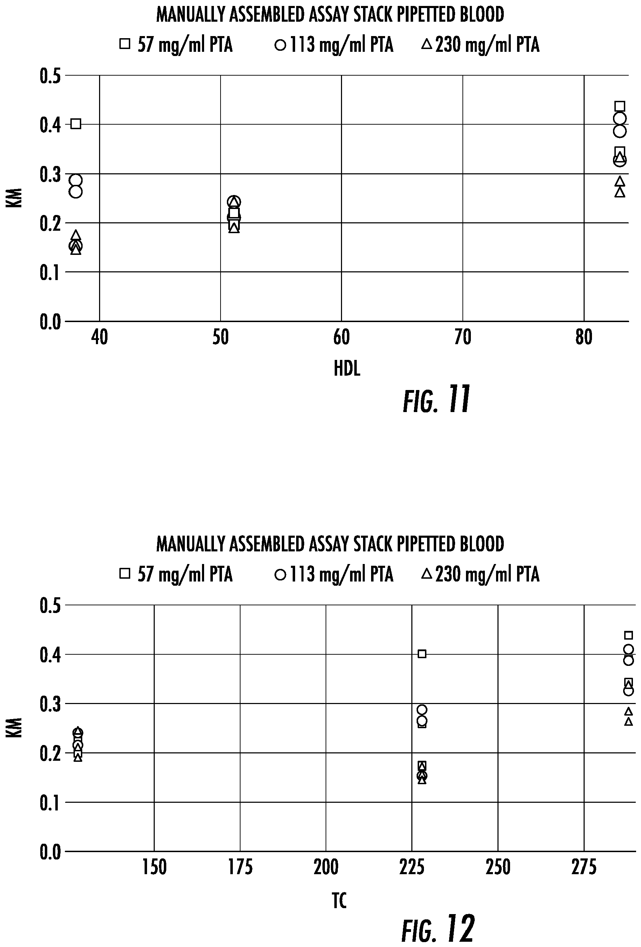

Various concentrations of a precipitating reagent (phosphotungstic acid) were added to the separation membrane of a manually assembled assay stack to determine the desired concentration for the best correlation between the signal measured (Kubelka-Munk intensity or KM) and the concentration of high density lipoprotein (HDL) present in blood fluid samples that were pipetted onto a separation membrane of an assay stack that also included a detection membrane. The separation membrane utilized was a Vivid^{®} GR polysulfone asymmetric membrane from Pall Corporation, while the detection membrane was a MMM polysulfone asymmetric membrane also from Pall Corporation. The concentrations of phosphotungstic acid tested were 57 milligrams/milliliter, 113 milligrams/milliliter, and 230 milligrams/milliliter. Assays were performed to determine the concentration of HDL and total cholesterol (TC) in the blood fluid sample based on the KM signal measured. The results are shown in FIGs. 11 and 12.

FIG. 11 shows a graph of high density lipoprotein concentration versus signal (Kubelka-Munk intensity or KM) expressed by a reagent for HDL in a detection membrane when the separation membrane contained varying levels of a first reagent for precipitating the non-high density lipoproteins (non-HDL) in a blood fluid sample for a manually assembled assay stack. Meanwhile, FIG. 12 shows a graph of total cholesterol concentration versus signal (Kubelka-Munk intensity or KM) expressed by a reagent for total cholesterol (TC) in a detection membrane when the separation membrane contained varying levels of a first reagent for precipitating the non-high density lipoproteins (non-HDL) in a blood fluid sample for a manually assembled assay stack.

As can be seen from FIG. 11, at low concentrations of the non-HDL precipitating reagent on the separation membrane (e.g., 57 milligrams/milliliter), the relationship between the HDL concentration and the signal (KM) is not monotonic over concentrations of HDL (e.g., about 38 milligrams per deciliter, about 51 milligrams per deciliter, and about 85 milligrams per deciliter), which indicates that the signal generated at the detection membrane is not generated exclusively by HDL. On the other hand, at high concentrations of the non-HDL precipitating reagent on the separation membrane (e.g., 230 milligrams/milliliter), the relationship between the HDL concentration and the signal (KM) is monotonic and linear, indicating that the signal generated at the detection membrane is caused primarily by the HDL. For comparison, as shown in FIG. 12, the total cholesterol was also measured at the detection membrane. The relationship between the total cholesterol concentration and the signal (KM) is not monotonic, which shows that the assay stack arrangement of this embodiment of an assay device of the present disclosure is specific to HDL at optimized concentrations of the precipitating reagent in the separation membrane.

### EXAMPLE 2

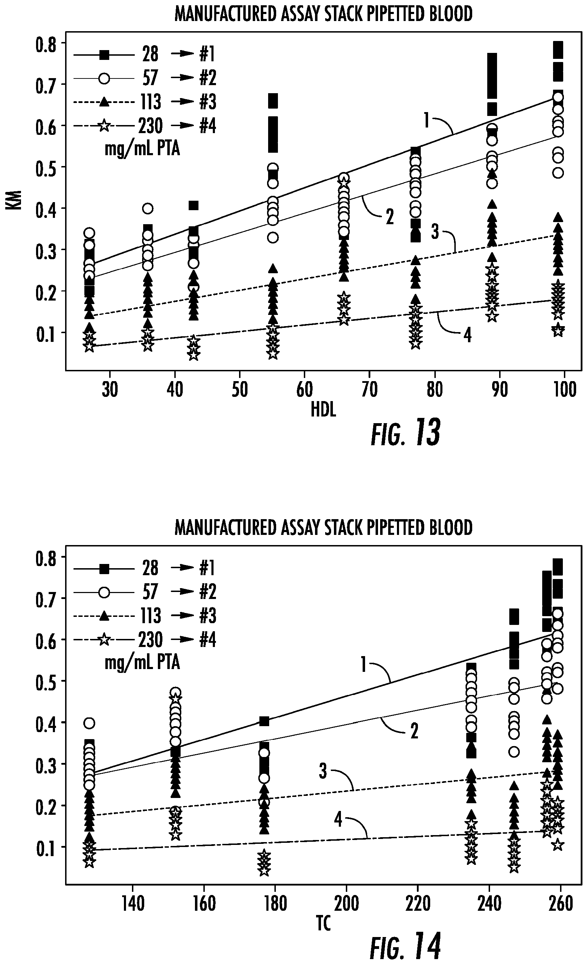

Various concentrations of a precipitating reagent (phosphotungstic acid) were added to the separation membrane of a manufactured assay stack to determine the desired concentration for the best correlation between the signal measured (Kubelka-Munk intensity or KM) and the concentration of high density lipoprotein (HDL) present in blood fluid samples that were pipetted onto a separation membrane of an assay stack that also included a detection membrane. The separation membrane utilized was a Vivid^{®} GR polysulfone asymmetric membrane from Pall Corporation, while the detection membrane was a MMM polysulfone asymmetric membrane also from Pall Corporation. The concentrations of phosphotungstic acid tested were 28 milligrams/milliliter, 57 milligrams/milliliter, 113 milligrams/milliliter, and 230 milligrams/milliliter. Assays were performed to determine the concentration of HDL, total cholesterol (TC), and non-HDL in the blood fluid samples based on the KM signal measured. The results are shown in FIGs. 13-15 and the table below.

| Phosphotungstic Acid Concentration (mg/mL) | r² vs. HDL (%) | r² vs. TC (%) | Coefficient of Variation in Measured Concentration using a KM fit (n=12) |
|---|---|---|---|
| 28 | 61.7 | 68.3 | 16.0 |
| 57 | 79.4 | 53.6 | 18.1 |
| 113 | 66.3 | 30.1 | 25.8 |
| 230 | 38.2 | 8.4 | 23.9 |

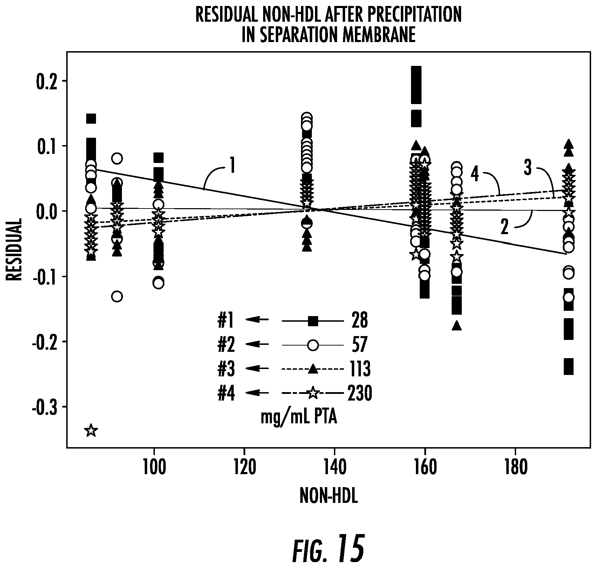

FIG. 13 shows a graph of high density lipoprotein concentration versus signal (Kubelka-Munk intensity or KM) expressed by a reagent for high density lipoprotein in a detection membrane when the separation membrane contained varying levels of a first reagent for precipitating the non-high density lipoproteins in a blood fluid sample for a manufactured assay stack. Further, FIG. 14 shows a graph of total cholesterol concentration versus signal (Kubelka-Munk intensity or KM) expressed by a reagent for total cholesterol in a detection membrane when the separation membrane contained varying levels of a first reagent for precipitating the non-high density lipoproteins in a blood fluid sample for a manufactured assay stack. Meanwhile, FIG. 15 shows a graph of residual non-high density lipoprotein concentration versus signal (Kubelka-Munk intensity or KM) expressed by a reagent for non-high density lipoprotein in a detection membrane when the separation membrane contained varying levels of the reagent for precipitating the non-high density lipoproteins in a blood fluid sample for a manufactured assay stack.

As can be seen from the table above and FIGs. 13-15, with the manufactured assay stacks containing the separation membrane and detection membrane contemplated by the present disclosure, concentrations of the precipitating agent that were greater than 28 milligrams/milliliter (e.g., 57 milligrams/milliliter, 113 milligrams/milliliter, and 230 milligrams/milliliter) were sufficient to fully precipitate the non-HDL in the blood fluid samples. This is evidenced by the monotonic relationship between the concentration of HDL and KM signal (see FIG. 13), the non-monotonic relationship between TC concentration and KM (see FIG. 14) and the correlation near zero for the difference between the linear fit and actual data versus the non-HDL concentration (see FIG. 15) for the concentrations greater than 28 milligrams/milliliter (see FIG. 15).

While various embodiments of the present disclosure have been described above, it should be understood that they have been presented by way of example only, and not by way of limitation. Likewise, the various diagrams may depict an example architectural or other configuration for the disclosure, which is done to aid in understanding the features and functionality that can be included in the disclosure.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. As examples of the foregoing, the term 'including' should be read to mean `including, without limitation,' `including but not limited to,' or the like; the term 'comprising' as used herein is synonymous with 'including,' 'containing,' or `characterized by,' and is inclusive or open-ended and does not exclude additional, unrecited elements or method steps; the term 'having' should be interpreted as 'having at least;' the term 'includes' should be interpreted as `includes but is not limited to;' the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the present disclosure, but instead as merely intended to highlight alternative or additional features that may or may not be utilized in a particular embodiment of the present disclosure.

Where a range of values is provided, it is understood that the upper and lower limit, and each intervening value between the upper and lower limit of the range is encompassed within the embodiments.

The indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage.

Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

While the present subject matter has been described in detail with respect to various specific example embodiments thereof, each example is provided by way of explanation, not limitation of the scope as defined by the appended claims. For instance, features illustrated or described as part of one embodiment can be used with another embodiment to yield a still further embodiment.

## Claims

1. An assay device for analyzing a blood fluid sample (814), wherein the assay device comprises:
a separation membrane (806) containing a first reagent (818) for precipitating a first protein (824) contained in the blood fluid sample, further wherein the separation membrane separates red blood cells (822) from the blood fluid sample, and the first reagent comprises a polyanionic non-high density lipoprotein binding reagent;
a detection membrane (812) configured to elicit a quantifiable response (844) in the presence of a second protein (816) present in the blood fluid sample, wherein the quantifiable response corresponds to an amount of the second protein present in the detection membrane, wherein the separation membrane concurrently precipitates the first protein and separates the red blood cells from the blood fluid sample that reaches the detection membrane; and
wherein the first reagent is present in the separation membrane at (a) a concentration ranging from about 35 milligrams/milliliter to about 250 milligrams/milliliter based on the wet weight of the first reagent present on the separation membrane, or (b) a concentration ranging from about 0.7 milligrams/square centimeter to about 10 milligrams/square centimeter based on the dry weight of the first reagent present on the separation membrane.

2. The assay device according to claim 1, wherein the first reagent is not immobilized on the separation membrane.

3. The assay device according to claim 1 or 2, wherein the first protein comprises a non-high density lipoprotein and/or the second protein comprises a high density lipoprotein.

4. The assay device according to any one of the preceding claims, wherein the separation membrane includes pores having a pore size ranging from about 0.1 micrometers to about 7.5 micrometers.

5. The assay device according to any one of the preceding claims, wherein the first reagent comprises a sulfonated polysaccharide, heparin, or phosphotungstic acid.

6. The assay device according to claim 5, wherein the first reagent further comprises a group II cation selected from the group of Mg, Mn, or Ca.

7. The assay device according to claim 6, wherein the first reagent comprises phosphotungstic acid and Mg.

8. The assay device according to any one of the preceding claims, wherein the separation membrane comprises a polysulfone, a polyethersulfone, a polyarylsulfone, a nylon, a nitrocellulose, cellulose filter paper, glass fiber, or a combination thereof.

9. The assay device according to any one of the preceding claims, wherein at least one component of the assay device is compressible, thereby allowing for an uncompressed state and a compressed state of the assay device and/or the assay device comprises a metering stack (224, 304, 504, 604, 802) configured to receive and distribute the blood fluid sample along a channel.

10. The assay device according to claim 9, wherein at least one component of the assay device is compressible, thereby allowing for an uncompressed state and a compressed state of the assay device and the assay device comprises a metering stack (224, 304, 504, 604, 802) configured to receive and distribute the blood fluid sample along a channel, and wherein a spacer material (225) is disposed between the metering stack and the separation membrane, wherein the spacer material provides a gap between the metering stack and the separation membrane that prevents the blood fluid sample from flowing from the metering stack to the separation membrane when the assay device is in the uncompressed state.

11. The assay device according to any one of the preceding claims, wherein the quantifiable response in the presence of a second protein present in the blood fluid sample comprises a color change of at least a portion of the detection membrane.

12. In-vitro use of the assay device according to any one of claims 1 to 12 for determining the concentration of the second protein present in the blood fluid sample.

13. A method of fabricating an assay device for analyzing a blood fluid sample (814), the method comprising:
Applying a first reagent (818) comprising a polyanionic non-high density lipoprotein binding reagent to a separation membrane (806), wherein the first reagent precipitates a first protein (824) contained in the blood fluid sample when the blood fluid sample contacts the separation membrane, further wherein the separation membrane is configured to separate red blood cells (822) from the blood fluid sample;
allowing the first reagent to dry on the separation membrane; and
positioning a detection membrane (812) adjacent the separation membrane, wherein the detection membrane is configured to elicit a quantifiable response (844) in the presence of a second protein (816) present in the blood fluid sample, wherein the quantifiable response corresponds to an amount of the second protein present in the detection membrane,
wherein the separation membrane is configured to concurrently precipitate the first protein and separate the red blood cells from the blood fluid sample that reaches the detection membrane, and
wherein the first reagent is present in the separation membrane at (a) a concentration ranging from about 35 milligrams/milliliter to about 250 milligrams/milliliter based on the wet weight of the first reagent present on the separation membrane, or (b) a concentration ranging from about 0.7 milligrams/square centimeter to about 10 milligrams/square centimeter based on the dry weight of the first reagent present on the separation membrane.

14. The method according to claim 13, wherein the first reagent comprises a sulfonated polysaccharide, heparin, or phosphotungstic acid and a group II cation selected from the group of Mg, Mn, or Ca.

15. The method according to claim 14, wherein the first reagent comprises phosphotungstic acid and Mg.

## Patentansprüche

1. Testvorrichtung zum Analysieren einer Blutflüssigkeitsprobe (814), wobei die Testvorrichtung umfasst:
eine Trennmembran (806), die ein erstes Reagens (818) zum Ausfällen eines ersten Proteins (824) enthält, das in der Blutflüssigkeitsprobe enthalten ist, wobei die Trennmembran ferner rote Blutzellen (822) von der Blutflüssigkeitsprobe trennt und das erste Reagens ein polyanionisches Lipoproteinbindungsreagens mit nicht hoher Dichte umfasst;
eine Nachweismembran (812), die so konfiguriert ist, dass sie in Gegenwart eines zweiten Proteins (816), das in der Blutflüssigkeitsprobe vorhanden ist, eine quantifizierbare Antwort (844) hervorruft, wobei die quantifizierbare Antwort einer Menge des zweiten Proteins entspricht, das in der Nachweismembran vorhanden ist, wobei die Trennmembran gleichzeitig das erste Protein ausfällt und die roten Blutzellen von der Blutflüssigkeitsprobe trennt, die die Nachweismembran erreicht; und
wobei das erste Reagens in der Trennmembran in (a) einer Konzentration im Bereich von etwa 35 Milligramm/Milliliter bis etwa 250 Milligramm/Milliliter basierend auf dem Nassgewicht des ersten Reagens, das auf der Trennmembran vorhanden ist, oder (b) in einer Konzentration im Bereich von etwa
0,7 Milligramm/Quadratzentimeter bis etwa
10 Milligramm/Quadratzentimeter basierend auf dem Trockengewicht des ersten Reagens, das auf der Trennmembran vorhanden ist, vorliegt.

2. Testvorrichtung nach Anspruch 1, wobei das erste Reagens nicht auf der Trennmembran immobilisiert ist.

3. Testvorrichtung nach Anspruch 1 oder 2, wobei das erste Protein ein Lipoprotein mit nicht hoher Dichte umfasst und/oder das zweite Protein ein Lipoprotein mit hoher Dichte umfasst.

4. Testvorrichtung nach einem der vorhergehenden Ansprüche, wobei die Trennmembran Poren mit einer Porengröße im Bereich von etwa 0,1 Mikrometer bis etwa 7,5 Mikrometer beinhaltet.

5. Testvorrichtung nach einem der vorhergehenden Ansprüche, wobei das erste Reagens ein sulfoniertes Polysaccharid, Heparin oder Phosphorwolframsäure umfasst.

6. Testvorrichtung nach Anspruch 5, wobei das erste Reagens ferner ein Kation der Gruppe II umfasst, das aus der Gruppe bestehend aus Mg, Mn oder Ca ausgewählt ist.

7. Testvorrichtung nach Anspruch 6, wobei das erste Reagens Phosphorwolframsäure und Mg umfasst.

8. Testvorrichtung nach einem der vorhergehenden Ansprüche, wobei die Trennmembran ein Polysulfon, ein Polyethersulfon, ein Polyarylsulfon, ein Nylon, eine Nitrocellulose, Cellulosefilterpapier, Glasfaser oder eine Kombination davon umfasst.

9. Testvorrichtung nach einem der vorhergehenden Ansprüche, wobei zumindest eine Komponente der Testvorrichtung komprimierbar ist, wodurch ein unkomprimierter Zustand und ein komprimierter Zustand der Testvorrichtung möglich ist, und/oder die Testvorrichtung einen Dosierstapel (224, 304, 504, 604, 802) umfasst, der so konfiguriert ist, dass er die Blutflüssigkeitsprobe empfängt und entlang eines Kanals verteilt.

10. Testvorrichtung nach Anspruch 9, wobei zumindest eine Komponente der Testvorrichtung komprimierbar ist, wodurch ein nicht komprimierter Zustand und ein komprimierter Zustand der Testvorrichtung ermöglicht wird, und die Testvorrichtung einen Dosierstapel (224, 304, 504, 604, 802) umfasst, der so konfiguriert ist, dass er die Blutflüssigkeitsprobe aufnimmt und entlang eines Kanals verteilt, und wobei ein Abstandshaltermaterial (225) zwischen dem Dosierstapel und der Trennmembran angeordnet ist, wobei das Abstandshaltermaterial einen Spalt zwischen dem Dosierstapel und der Trennmembran bereitstellt, der verhindert, dass die Blutflüssigkeitsprobe von dem Dosierstapel zu der Trennmembran fließt, wenn sich die Testvorrichtung in dem nicht komprimierten Zustand befindet.

11. Testvorrichtung nach einem der vorhergehenden Ansprüche, wobei die quantifizierbare Antwort in Gegenwart eines zweiten Proteins, das in der Blutflüssigkeitsprobe vorhanden ist, eine Farbänderung von zumindest einem Teil der Nachweismembran umfasst.

12. In-vitro-Verwendung der Testvorrichtung nach einem der Ansprüche 1 bis 12 zum Ermitteln der Konzentration des zweiten Proteins, das in der Blutflüssigkeitsprobe vorliegt.

13. Verfahren zur Herstellung einer Testvorrichtung zum Analysieren einer Blutflüssigkeitsprobe (814), wobei das Verfahren umfasst:
Aufbringen eines ersten Reagens (818), das ein polyanionisches Lipoproteinbindungsreagens mit nicht hoher Dichte umfasst, auf eine Trennmembran (806), wobei das erste Reagens ein erstes Protein (824) ausfällt, das in der Blutflüssigkeitsprobe enthalten ist, wenn die Blutflüssigkeitsprobe mit der Trennmembran in Kontakt kommt, wobei die Trennmembran ferner so konfiguriert ist, dass sie rote Blutzellen (822) von der Blutflüssigkeitsprobe trennt;
Ermöglichen des Trocknens des ersten Reagens auf der Trennmembran; und
Positionieren einer Nachweismembran (812) angrenzend an die Trennmembran, wobei
die Nachweismembran so konfiguriert ist, dass sie in Gegenwart eines zweiten Proteins (816), das in der Blutflüssigkeitsprobe vorhanden ist, eine quantifizierbare Antwort (844) hervorruft, wobei die quantifizierbare Antwort einer Menge des zweiten Proteins entspricht, das in der Nachweismembran vorhanden ist,
wobei die Trennmembran so konfiguriert ist, dass sie gleichzeitig das erste Protein ausfällt und die roten Blutzellen von der Blutflüssigkeitsprobe trennt, die die Nachweismembran erreicht, und
wobei das erste Reagens in der Trennmembran in (a) einer Konzentration im Bereich von etwa 35 Milligramm/Milliliter bis etwa 250 Milligramm/Milliliter basierend auf dem Nassgewicht des ersten Reagens, das auf der Trennmembran vorhanden ist, oder (b) in einer Konzentration im Bereich von etwa
0,7 Milligramm/Quadratzentimeter bis etwa
10 Milligramm/Quadratzentimeter basierend auf dem Trockengewicht des ersten Reagens, das auf der Trennmembran vorhanden ist, vorliegt.

14. Verfahren nach Anspruch 13, wobei das erste Reagens ein sulfoniertes Polysaccharid, Heparin oder Phosphorwolframsäure und ein Kation der Gruppe II, ausgewählt aus der Gruppe bestehend aus Mg, Mn oder Ca, umfasst.

15. Verfahren nach Anspruch 14, wobei das erste Reagens Phosphorwolframsäure und Mg umfasst.

## Revendications

1. Dispositif de dosage pour analyser un échantillon de fluide sanguin (814), dans lequel le dispositif de dosage comprend :
une membrane de séparation (806) contenant un premier réactif (818) pour précipiter une première protéine (824) contenue dans l'échantillon de fluide sanguin, dans lequel en outre la membrane de séparation sépare les globules rouges (822) de l'échantillon de fluide sanguin, et le premier réactif comprend un réactif polyanionique de liaison de lipoprotéine de non haute densité ;
une membrane de détection (812) configurée pour déclencher une réponse quantifiable (844) en présence d'une deuxième protéine (816) présente dans l'échantillon de fluide sanguin, dans lequel la réponse quantifiable correspond à une quantité de la deuxième protéine présente dans la membrane de détection, dans lequel, simultanément, la membrane de séparation précipite la première protéine et sépare les globules rouges de l'échantillon de fluide sanguin qui atteint la membrane de détection ; et
dans lequel le premier réactif est présent dans la membrane de séparation à (a) une concentration allant d'environ 35 milligrammes/millilitre à environ 250 milligrammes/millilitre sur la base du poids humide du premier réactif présent sur la membrane de séparation, ou (b) une concentration allant d'environ 0,7 milligramme/cm² à environ 10 milligrammes/cm² sur la base du poids à sec du premier réactif présent sur la membrane de séparation.

2. Dispositif de dosage selon la revendication 1, dans lequel le premier réactif n'est pas immobilisé sur la membrane de séparation.

3. Dispositif de dosage selon la revendication 1 ou 2, dans lequel la première protéine comprend une lipoprotéine de non haute densité et/ou la deuxième protéine comprend une lipoprotéine de haute densité.

4. Dispositif de dosage selon l'une quelconque des revendications précédentes, dans lequel la membrane de séparation comprend des pores ayant une taille de pores allant d'environ 0,1 micromètre à environ 7,5 micromètres.

5. Dispositif de dosage selon l'une quelconque des revendications précédentes, dans lequel le premier réactif comprend un polysaccharide sulfoné, de l'héparine ou de l'acide phosphotungstique.

6. Dispositif de dosage selon la revendication 5, dans lequel le premier réactif comprend en outre un cation du groupe II choisi dans le groupe de Mg, Mn ou Ca.

7. Dispositif de dosage selon la revendication 6, dans lequel le premier réactif comprend de l'acide phosphotungstique et du Mg.

8. Dispositif de dosage selon l'une quelconque des revendications précédentes, dans lequel la membrane de séparation comprend une polysulfone, une polyéthersulfone, une polyarylsulfone, un nylon, une nitrocellulose, un papier filtre cellulosique, une fibre de verre ou une combinaison de ceux-ci.

9. Dispositif de dosage selon l'une quelconque des revendications précédentes, dans lequel au moins un composant du dispositif de dosage peut être comprimé, permettant ainsi un état non comprimé et un état comprimé du dispositif de dosage et/ou le dispositif de dosage comprend un empilement de mesure (224, 304, 504, 604, 802) configuré pour recevoir et distribuer l'échantillon de fluide sanguin le long d'un canal.

10. Dispositif de dosage selon la revendication 9, dans lequel au moins un composant du dispositif de dosage peut être comprimé, permettant ainsi un état non comprimé et un état comprimé du dispositif de dosage et le dispositif de dosage comprend un empilement de mesure (224, 304, 504, 604, 802) configuré pour recevoir et distribuer l'échantillon de fluide sanguin le long d'un canal, et dans lequel un matériau d'espacement (225) est disposé entre l'empilement de mesure et la membrane de séparation, dans lequel le matériau d'espacement fournit un écart entre l'empilement de mesure et la membrane de séparation qui empêche l'échantillon de fluide sanguin de s'écouler de l'empilement de mesure vers la membrane de séparation lorsque le dispositif de dosage est dans l'état non comprimé.

11. Dispositif de dosage selon l'une quelconque des revendications précédentes, dans lequel la réponse quantifiable en présence d'une deuxième protéine présente dans l'échantillon de fluide sanguin comprend un changement de couleur d'au moins une partie de la membrane de détection.

12. Utilisation in vitro du dispositif de dosage selon l'une quelconque des revendications 1 à 12 pour déterminer la concentration de la deuxième protéine présente dans l'échantillon de fluide sanguin.

13. Procédé de fabrication d'un dispositif de dosage pour analyser un échantillon de fluide sanguin (814), le procédé comprenant :
l'application d'un premier réactif (818) comprenant un réactif polyanionique de liaison de lipoprotéine de non haute densité à une membrane de séparation (806), dans lequel le premier réactif précipite une première protéine (824) contenue dans l'échantillon de fluide sanguin lorsque l'échantillon de fluide sanguin entre en contact avec la membrane de séparation, dans lequel la membrane de séparation est en outre configurée pour séparer les globules rouges (822) de l'échantillon de fluide sanguin ;
le fait de laisser le premier réactif sécher sur la membrane de séparation ; et
le positionnement d'une membrane de détection (812) adjacente à la membrane de séparation, dans lequel la membrane de détection est configurée pour déclencher une réponse quantifiable (844) en présence d'une deuxième protéine (816) présente dans l'échantillon de fluide sanguin, dans lequel la réponse quantifiable correspond à une quantité de la deuxième protéine présente dans la membrane de détection,
dans lequel, la membrane de séparation est configurée pour, simultanément, précipiter la première protéine et séparer les globules rouges de l'échantillon de fluide sanguin qui atteint la membrane de détection, et
dans lequel le premier réactif est présent dans la membrane de séparation à (a) une concentration allant d'environ 35 milligrammes/millilitre à environ 250 milligrammes/millilitre sur la base du poids humide du premier réactif présent sur la membrane de séparation, ou (b) une concentration allant d'environ 0,7 milligramme/cm² à environ 10 milligrammes/cm² sur la base du poids à sec du premier réactif présent sur la membrane de séparation.

14. Procédé selon la revendication 13, dans lequel le premier réactif comprend un polysaccharide sulfoné, de l'héparine ou de l'acide phosphotungstique et un cation du groupe II choisi dans le groupe de Mg, Mn ou Ca.

15. Procédé selon la revendication 14, dans lequel le premier réactif comprend de l'acide phosphotungstique et du Mg.
